(19) Europäisches Patentamt / European Patent Office / Office européen des brevets

(11) **EP 3 545 571 B1**

(12) **EUROPEAN PATENT SPECIFICATION**

(45) Date of publication and mention
of the grant of the patent:
**05.01.2022 Bulletin 2022/01**

(21) Application number: **17873627.8**

(22) Date of filing: **24.11.2017**

(51) Int Cl.:
***C23C 14/20*** *(2006.01)*       ***A61B 5/296*** *(2021.01)*
***A61B 5/24*** *(2021.01)*

(86) International application number:
**PCT/SG2017/050582**

(87) International publication number:
**WO 2018/097801 (31.05.2018 Gazette 2018/22)**

(54) **STRETCHABLE ELECTRODE AND METHOD OF FORMING THE SAME**

DEHNBARE ELEKTRODE UND VERFAHREN ZUR HERSTELLUNG DERSELBEN

ÉLECTRODE EXTENSIBLE ET SON PROCÉDÉ DE FORMATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.11.2016 SG 10201609888R**

(43) Date of publication of application:
**02.10.2019 Bulletin 2019/40**

(73) Proprietor: **Nanyang Technological University
Singapore 639798 (SG)**

(72) Inventors:
• **LIU, Zhiyuan**
  **Singapore 639798 (SG)**
• **CHEN, Xiaodong**
  **Singapore 639798 (SG)**

(74) Representative: **Viering, Jentschura & Partner
mbB
Patent- und Rechtsanwälte
Am Brauhaus 8
01099 Dresden (DE)**

(56) References cited:
KR-A- 20160 084 171       US-A1- 2015 345 025
US-A1- 2016 213 269

• ANNA CYGANOWSKI ET AL: "Stretchable electrodes for neuroprosthetic interfaces", 2013 IEEE SENSORS, IEEE, 28 October 2012 (2012-10-28), pages 1-4, XP032308943, ISSN: 1930-0395, DOI: 10.1109/ICSENS.2012.6411503
• BOWDEN N. ET AL.: 'Spontaneous formation of ordered structures in thin films of metals supported on an elastomeric polymer' NATURE vol. 393, 14 May 1998, pages 146 - 149, XP055508309
• WEI K. ET AL.: 'Systematic investigation of the benchtop surface wrinkling process by corona discharge' RSC ADV. vol. 4, 31 October 2014, pages 59122 - 59129, XP055508308
• WANG Y. ET AL.: 'Stretchable Thin Film Materials: Fabrication, Application, and Mechanics' J. ELECTRON. PACKAG vol. 138, no. 2, 18 April 2016, pages 020801 - 01 -22, XP055508310
• TANG J. ET AL.: 'Highly Stretchable Electrodes on Wrinkled Polydimethylsiloxane Substrates' SCIENTIFIC REPORTS vol. 5, 20 November 2015, pages 1 - 9, XP055508311
• ARAFAT Y. ET AL.: 'Super-stretchable metallic interconnects on polymer with a linear strain of up to 100%' APPLIED PHYSICS LETTERS vol. 107, no. 8, 28 August 2015, page 081906, XP012199981

**Description**

**CROSS-REFERENCE TO RELATED APPLICATION**

This application claims the benefit of priority of Singapore application No. 10201609888R filed on November 24, 2016.

**TECHNICAL FIELD**

Various aspects of this disclosure relate to a stretchable electrode. Various aspects of this disclosure relate to a method of forming a stretchable electrode.

**BACKGROUND**

In recent years, there is a paradigm shift in the research field from silicon-based rigid electronics to bio-materials/elastic materials-based deformable electronics. The underlying driving force is the pursuit for more human-friendly electronics and greater human-machine integration to further facilitate daily life and enhance human power.

Under this direction, soft implantable devices, flexible electronic interfaces, smart sensors for Internet of Things (IoT), wearable diagnosis sensors, flexible batteries, soft robotics, and other stretchable devices are undergoing rapid development. In many of such flexible/stretchable devices, stretchable conductors (i.e. conductors that are able to exhibit conductivity even under large mechanical deformation (> 30% strain)) are required in order to achieve the desired functions.

However, there is hardly any intrinsically stretchable fully conductive material in nature. Several strategies have been proposed to fabricate the stretchable fully conductive materials by combining an elastic polymer with fully conductive materials, such as metal and carbon. Nevertheless, such a direct combination may result in poor interfacial adhesion, especially for a conductive metal layer (alternatively referred to as metal film) on an elastic substrate, because of the totally different physical and chemical properties of organic polymer and inorganic conductive materials. US2015/0345025 A1 discloses deposition of a gold thin film on amine functionalized polydimethylsiloxane (PDMS). Further relevant prior art is disclosed in the document BOWDEN N. ET AL.: "Spontaneous formation of ordered structures in thin films of metals supported on an elastomeric polymer",NATURE, vol. 393, 14 May 1998 (1998-05-14), pages 146-149.

Adhesion is vital both for the subsequent conductor encapsulation process (which typically involves multi-mask photolithography or a peel-off process demanding good adhesion of the metal film on elastic substrate) as well as for practical use, such as in interface signal monitoring which inevitably involves severe interface friction. If the metal film is not able to adhere well to the elastic substrate, the stretchable conductor may be unable to go through the encapsulation process or may fail in the short term in practical applications. As such, achieving high adhesion and high stretchability simultaneously for stretchable electronics is a big challenge.

**SUMMARY**

The present invention is defined by the appended claims. Various embodiments provide a method of forming a stretchable electrode. The method includes providing a non-fully cured sample on a substrate. The method also includes depositing a metal on the non-fully cured sample via thermal evaporation to form the stretchable electrode. The non-fully cured sample is uncured or is partially cured for a duration of less than 30 minutes. The partially cured sample is formed by heating at a temperature of less than 70 °C. The stretchable electrode includes a base layer, a wrinkled layer, a plurality of pillars extending from the base layer to the wrinkled layer, and a metal film on the wrinkled layer, the metal film including the metal. The base layer, the wrinkled layer, and the plurality of pillars include polydimethylsiloxane.

Various embodiments may provide a stretchable electrode. The stretchable electrode includes a base layer. The stretchable electrode also includes a wrinkled layer. The stretchable electrode further includes a plurality of pillars extending from the base layer to the wrinkled layer. The stretchable electrode additionally includes a metal film on the wrinkled layer, the metal film including a metal. The base layer, the wrinkled layer, and the plurality of pillars include polydimethylsiloxane.

**BRIEF DESCRIPTION OF THE DRAWINGS**

The invention will be better understood with reference to the detailed description when considered in conjunction with the non-limiting examples and the accompanying drawings, in which:

FIG. 1 is a schematic illustrating a method of forming a stretchable electrode according to various embodiments.

FIG. 2 is a general illustration of a stretchable electrode according to various embodiments.

FIG. 3A shows (top) a plurality of gold clusters/nanoparticles being evaporated onto non-fully cured polydimethylsiloxane (PDMS) on a rigid backing, and (bottom) evaporated gold clusters/nanoparticles being received by the non-fully cured polydimethylsiloxane (PDMS) according to various embodiments.

FIG. 3B is a schematic illustrating physical models of heat transfer and viscous flowing occurring during the dynamic process of gold deposition onto non-fully cured polydimethylsiloxane (PDMS) according to various embodiments.

FIG. 3C is a schematic showing simulation results of corresponding temperature distribution and pressure distribution during viscous flow (low viscous flow on the left and high viscous flow on the right) when gold is deposited onto non-fully cured polydimethylsiloxane (PDMS) according to various embodiments.

FIG. 3D is a schematic showing a resulting structure of the stretchable electrode according to various embodiments.

FIG. 4A shows a cross-sectional scanning electron microscopy (SEM) image of the self-stripping double-layer structure according to various embodiments, with the insets showing optical images of the front view and back view of the sample according to various embodiments.

FIG. 4B shows a magnified cross-sectional scanning electron microscopy (SEM) image of the self-stripping double-layer structure according to various embodiments shown in FIG. 4A.

FIG. 4C shows another magnified cross-sectional scanning electron microscopy (SEM) image of the self-stripping double-layer structure according to various embodiments shown in FIG. 4A.

FIG. 4D shows yet another magnified cross-sectional scanning electron microscopy (SEM) image of the self-stripping double-layer structure according to various embodiments shown in FIG. 4A.

FIG. 4E shows a scanning electron microscopy (SEM) image of the gold film having the wrinkled structure according to various embodiments.

FIG. 4F shows an atomic force microscopy (AFM) image of the gold film having the wrinkled structure according to various embodiments.

FIG. 4G is an image showing the detailed morphology of the gold film according to various embodiments.

FIG. 4H is another image showing the detailed morphology of the gold film according to various embodiments.

FIG. 5A shows a planar surface electron microscopy (SEM) image formed from a sample pre-cured for a duration of 15 minutes and having a ratio of cross linker to monomer of 1 : 10 according to various embodiments.

FIG. 5B shows a planar surface electron microscopy (SEM) image formed from a sample pre-cured for a duration of 20 minutes and having a ratio of cross linker to monomer of 1 : 10 according to various embodiments.

FIG. 5C shows a planar surface electron microscopy (SEM) image formed from a sample pre-cured for a duration of 25 minutes and having a ratio of cross linker to monomer of 1 : 10 according to various embodiments.

FIG. 5D shows a planar surface electron microscopy (SEM) image formed from a sample pre-cured for a duration of 15 minutes and having a ratio of cross linker to monomer of 1 : 20 according to various embodiments.

FIG. 5E shows a planar surface electron microscopy (SEM) image formed from a sample pre-cured for a duration of 20 minutes and having a ratio of cross linker to monomer of 1 : 20 according to various embodiments.

FIG. 5F shows a planar surface electron microscopy (SEM) image formed from a sample pre-cured for a duration of 25 minutes and having a ratio of cross linker to monomer of 1 : 20 according to various embodiments.

FIG. 6A shows an atomic force microscopy (AFM) image of a wrinkled film according to various embodiments.

FIG. 6B shows another atomic force microscopy (AFM) image of the wrinkled film shown in

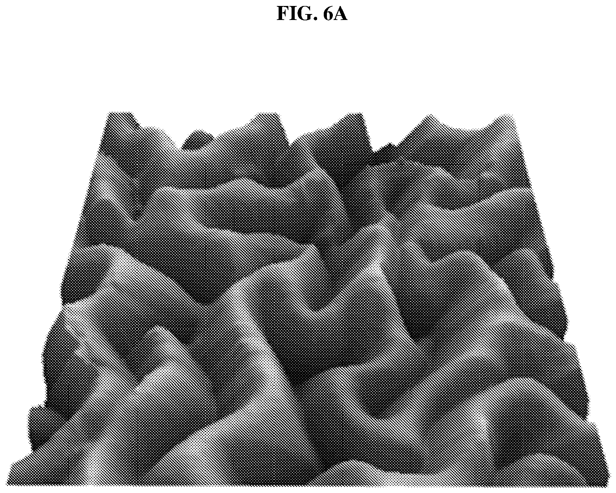

FIG. 6A according to various embodiments.

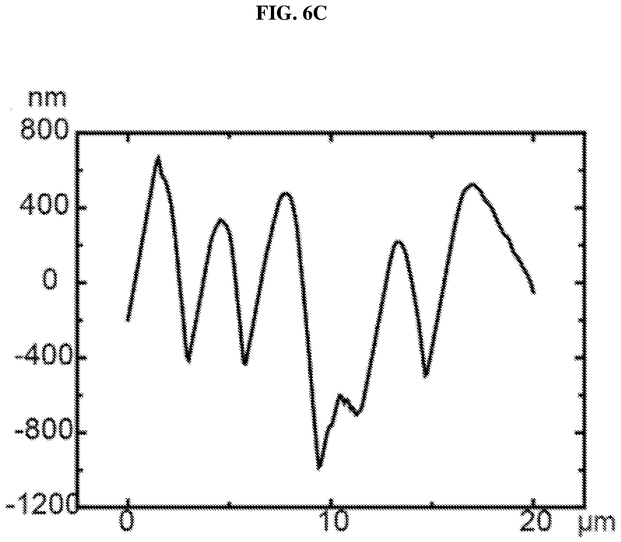

FIG. 6C is a plot of amplitude of the wrinkles in nanometers or nm) as a function of distance along the surface (in micrometers or $\mu$m) of the film shown in FIGS. 6A-B according to various embodiments.

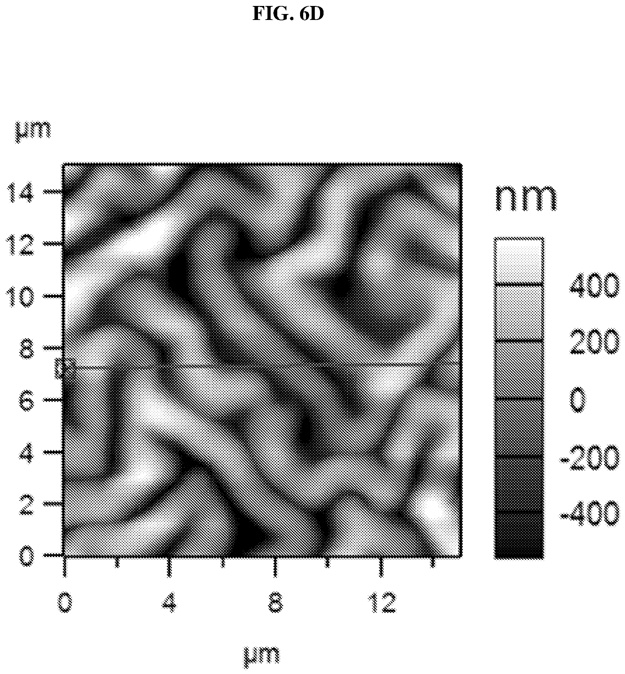

FIG. 6D shows an atomic force microscopy (AFM) image of another wrinkled film according to various embodiments.

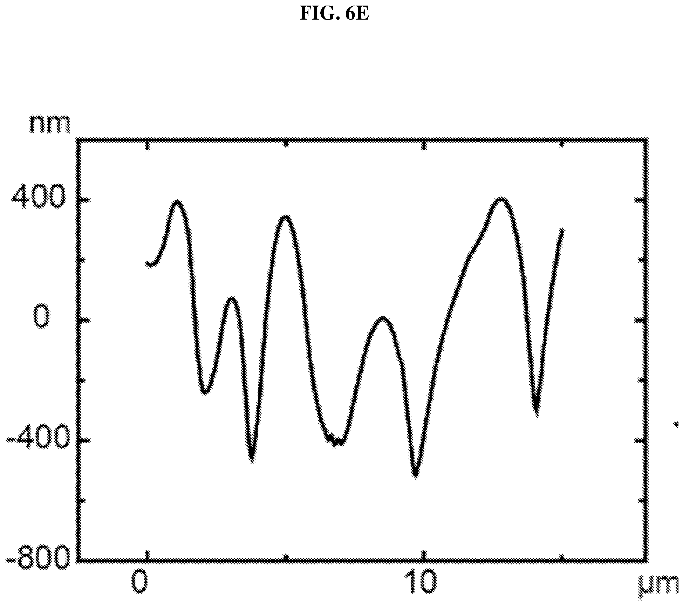

FIG. 6E is a plot of amplitude of the wrinkles in nanometers or nm) as a function of distance along the surface (in micrometers or $\mu$m) of the film shown in FIG. 6D according to various embodiments.

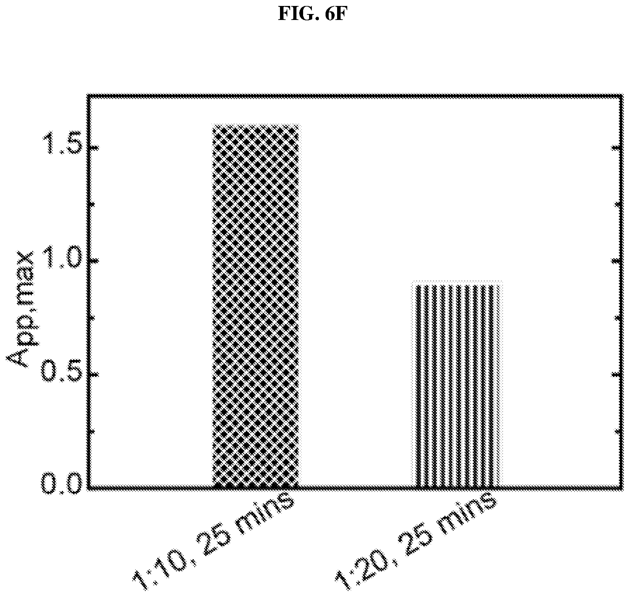

FIG. 6F is a plot of maximum amplitude (in micrometers or $\mu$m) comparing a first film formed with a ratio of cross linker to monomer of 1 : 10 and pre-cured for a duration of 25 minutes before deposition, and a second film formed with a ratio of cross linker to monomer of 1 : 20 and pre-cured for a duration of 25 minutes before deposition according to various embodiments.

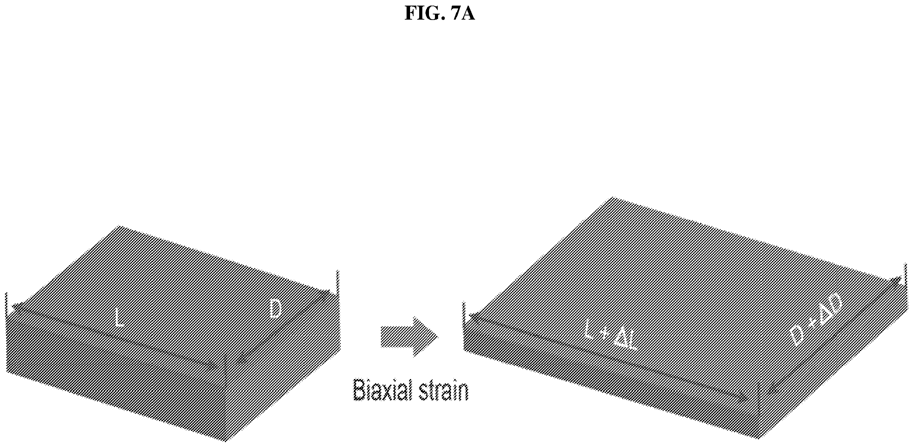

FIG. 7A shows a schematic illustrating the pre-biaxial strain method.

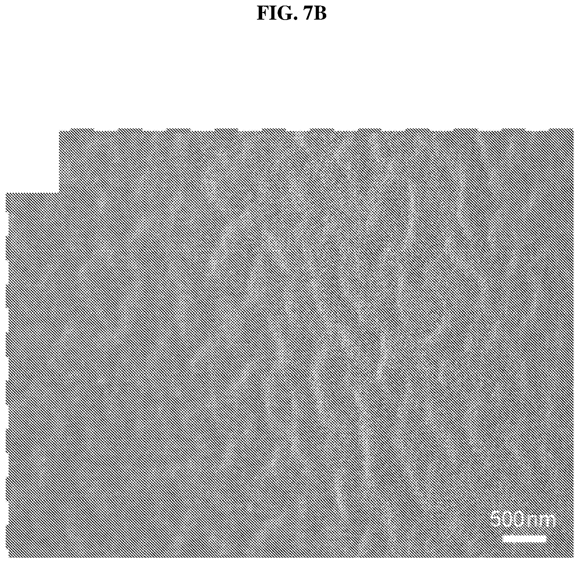

FIG. 7B shows a scanning electron microscopy (SEM) image of the film prepared by the pre-biaxial strain method.

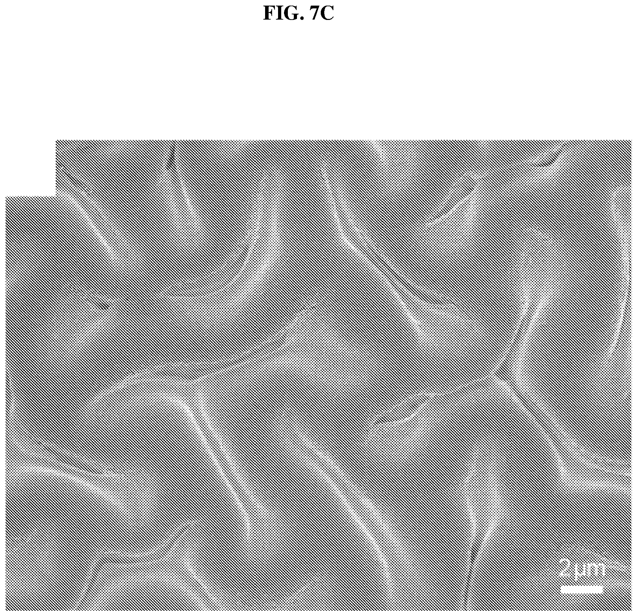

FIG. 7C shows a scanning electron microscopy (SEM) image of the film prepared by the method according to various embodiments.

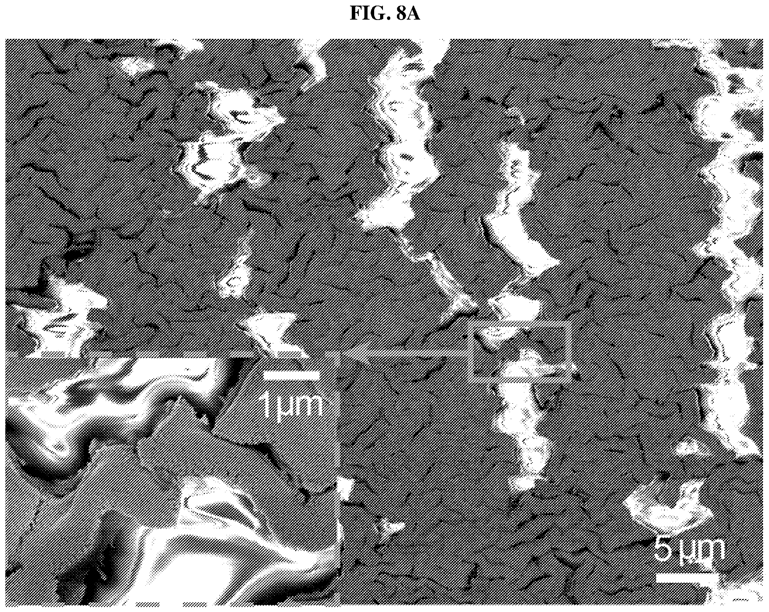

FIG. 8A shows a scanning electron microscopy (SEM) image of the stretchable electrode according to various embodiments under tensile strain of 50%, while the inset shows a magnified image of the upheaval blocks remain connected.

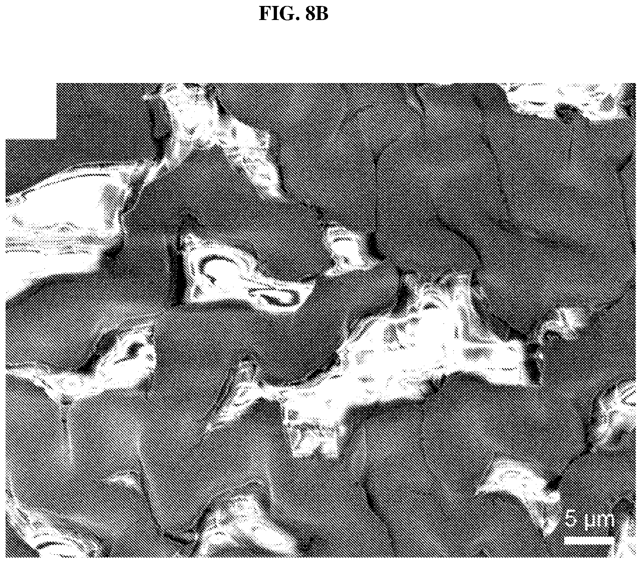

FIG. 8B shows a further scanning electron microscopy (SEM) image of the stretchable electrode according to various embodiments illustrating the connecting effect by twisting of the upheavals.

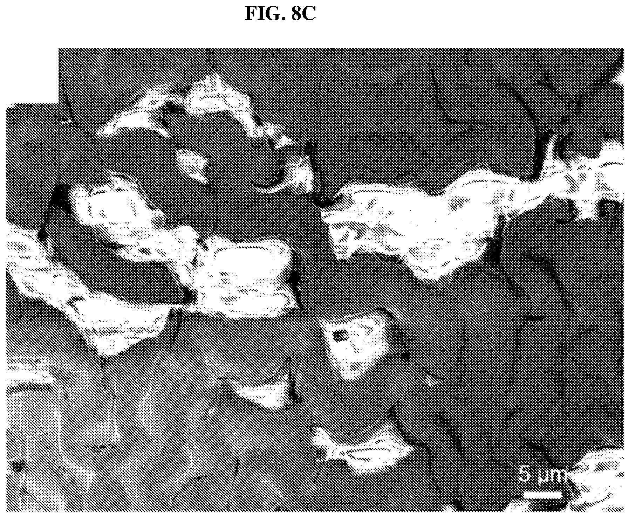

FIG. 8C shows another scanning electron microscopy (SEM) image of the stretchable electrode according to various embodiments illustrating the connecting effect by twisting of the upheavals.

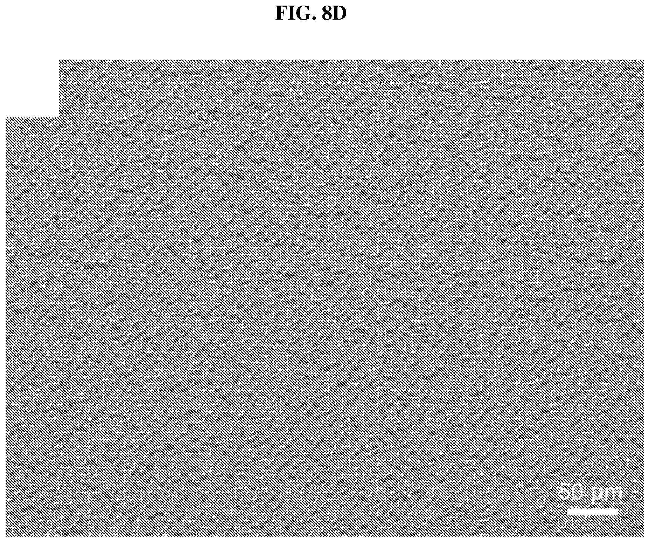

FIG. 8D shows a scanning electron microscopy (SEM) image of the stretchable electrode according to various embodiments under tensile strain of 30%.

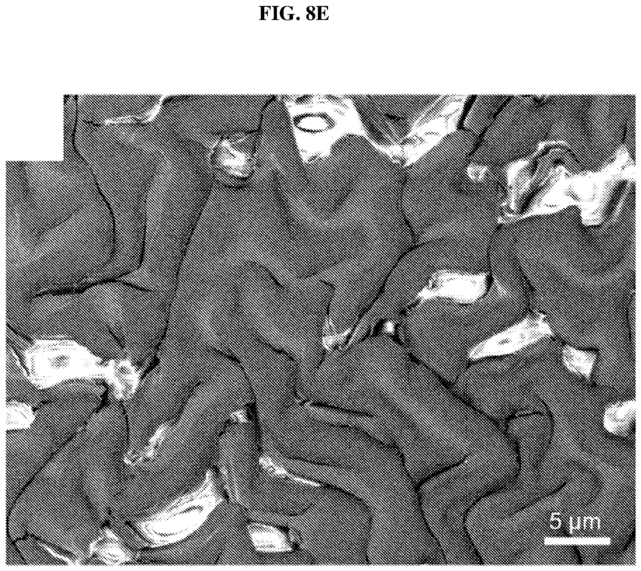

FIG. 8E shows a magnified image of the stretchable electrode shown in FIG. 8D according to various embodiments.

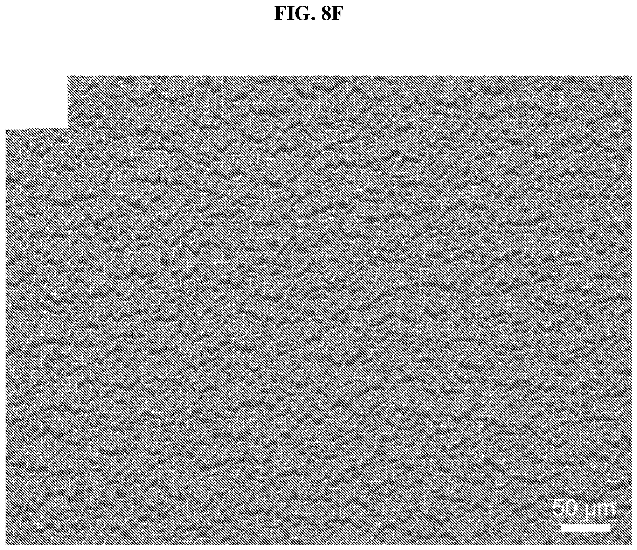

FIG. 8F shows a scanning electron microscopy (SEM) image of the stretchable electrode according to various embodiments under tensile strain of 30%.

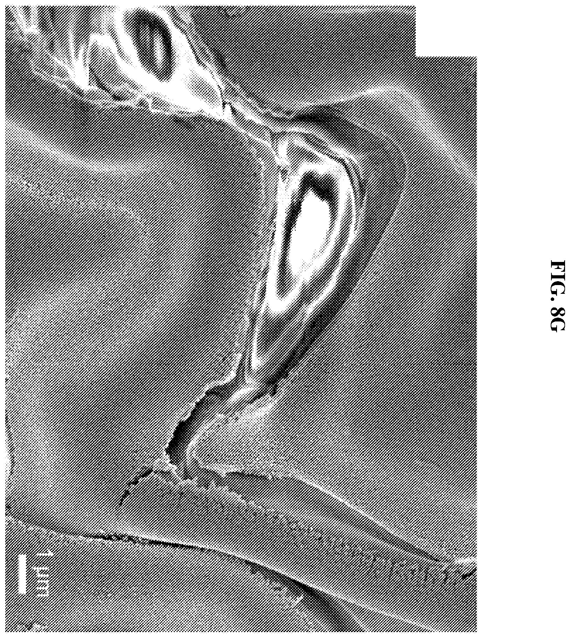

FIG. 8G shows a magnified image of the stretchable electrode shown in FIG. 8F according to various embodiments.

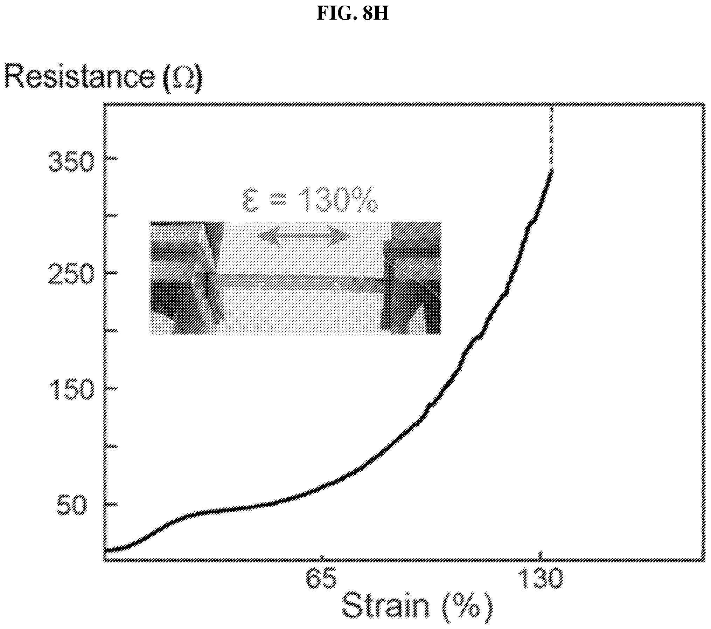

FIG. 8H is a plot of resistance (in ohms or $\Omega$) as a function of strain (in percent or %) showing the resistance change of the stretchable electrode according to various embodiments under varying strain, with the inset showing an optical image of the test setup.

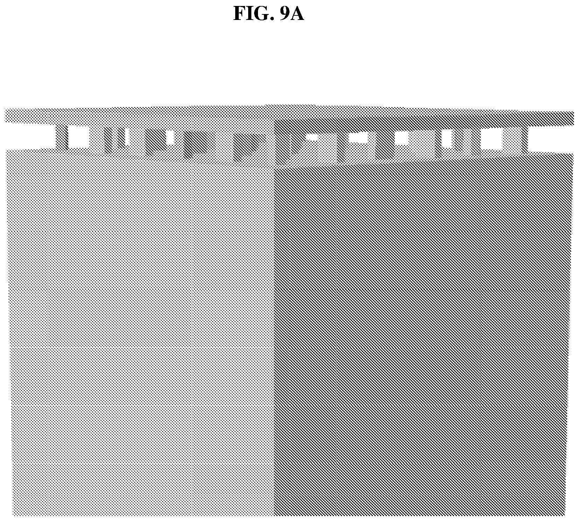

FIG. 9A is a schematic showing a finite element method (FEM) investigating the strain redistribution mechanism behind the stretchability of the stretchable electrode according to various embodiments.

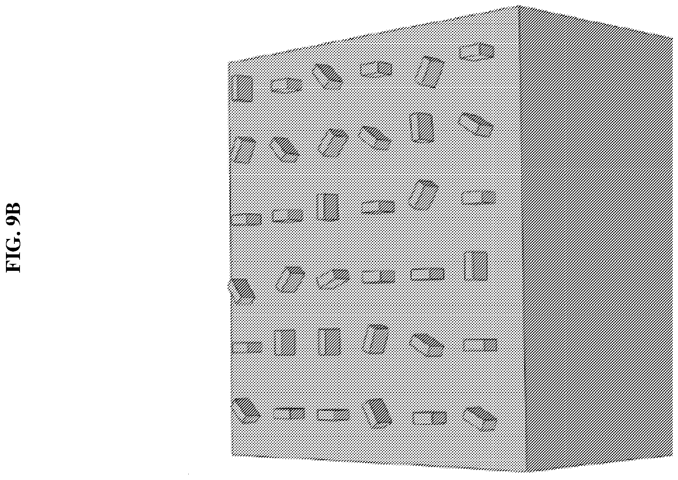

FIG. 9B is a schematic showing the finite element method (FEM) includes randomly oriented micro pillars in the simulation of the stretchable electrode according to various embodiments.

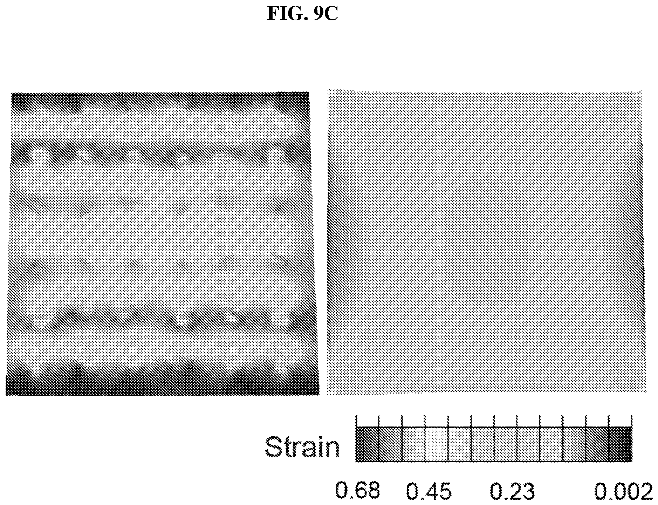

FIG. 9C shows the simulation results of the finite element method (FEM) investigating the strain redistribution mechanism behind the stretchability of the stretchable electrode according to various embodiments.

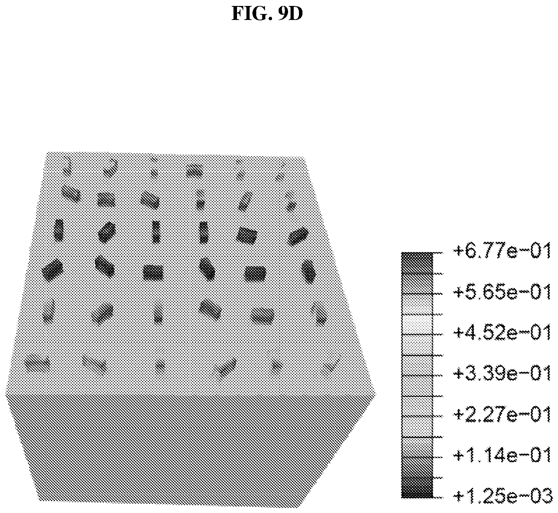

FIG. 9D shows the simulation of strain regulation effect induced by the micro pillars according to various embodiments.

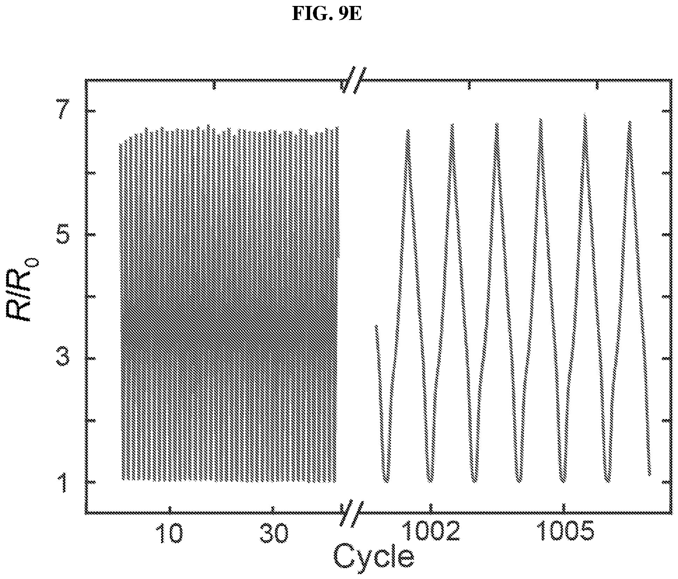

FIG. 9E is a plot of fractional change of resistance as a function of the number of cycles illustrating the resistance change of the stretchable electrode according to various embodiments under cyclic tensile strain.

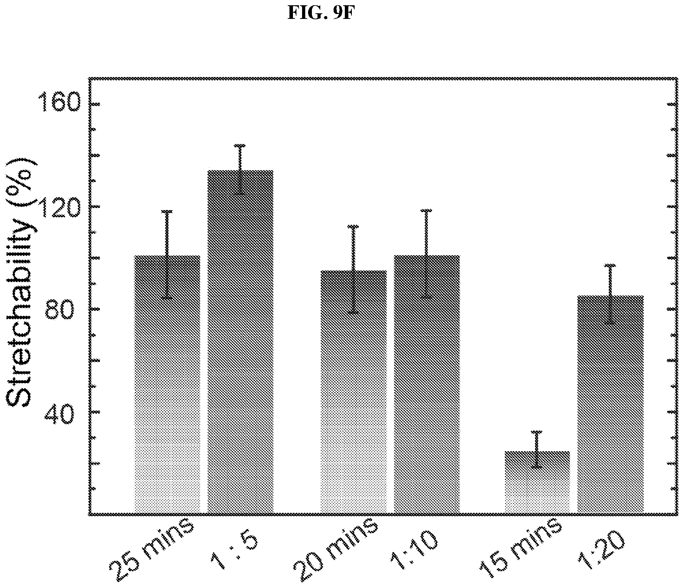

FIG. 9F is a plot of stretchability (in percent or %) as a function of pre-curing time / ratio of cross linker to monomer used according to various embodiments.

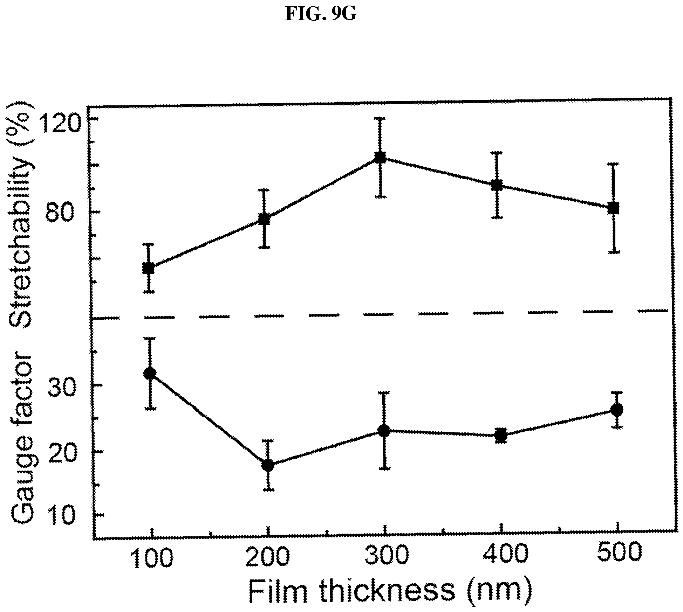

FIG. 9G is a plot of stretchability (in percent or %) /gauge factor as a function of film thickness (in nanometers or nm) according to various embodiments.

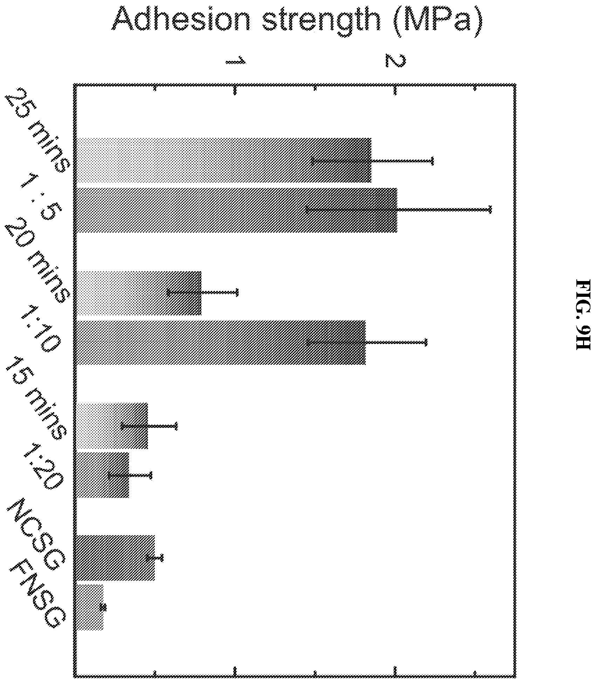

FIG. 9H is a plot of adhesion strength (in megaPascals or MPa) as a function of pre-curing time / ratio of cross linker to monomer used according to various embodiments.

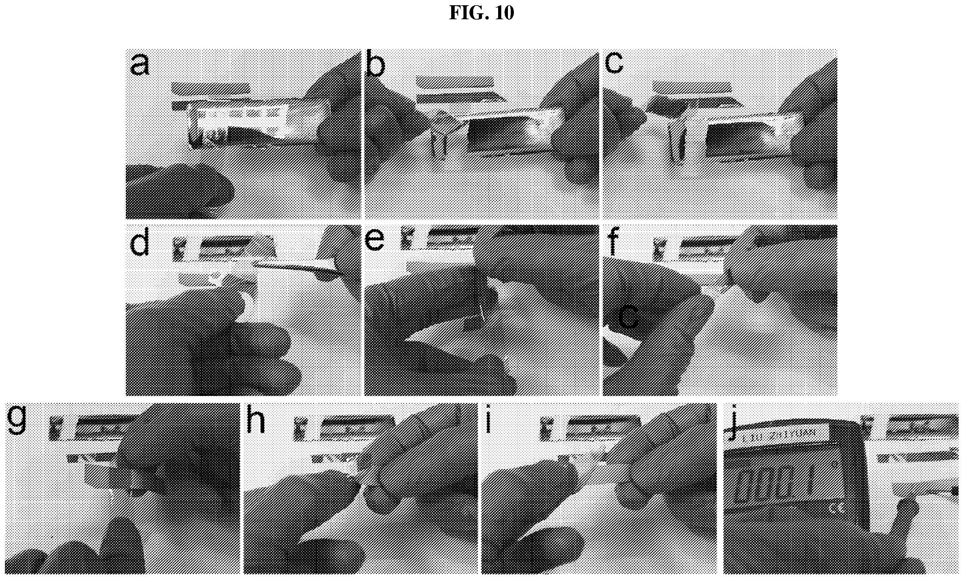

FIG. 10 shows (a) an image showing adhering a Kapton tape to a flat non-stretchable gold film, (b) an image showing peeling the Kapton tape from the flat non-stretchable gold film, (c) an image showing a portion of the flat non-stretchable gold film being peeled off, (d) an image showing adhering a Kapton tape to a normal crack-based stretchable gold film, (e) an image showing peeling the Kapton tape from the normal crack-based stretchable gold film, (f) an image showing a portion of the normal crack-based stretchable gold film being peeled off, (g) an image of adhering a Kapton tape to a stretchable electrode according to various embodiments, (h) an image showing peeling of the Kapton tape from the stretchable electrode according to various embodiments, (i) an image showing a portion of the stretchable electrode according to various embodiments being peeled off, and (j) an image of a multimeter to measure the electrical conductivity of the stretchable electrode according to various embodiments after the peel test in (g) - (i).

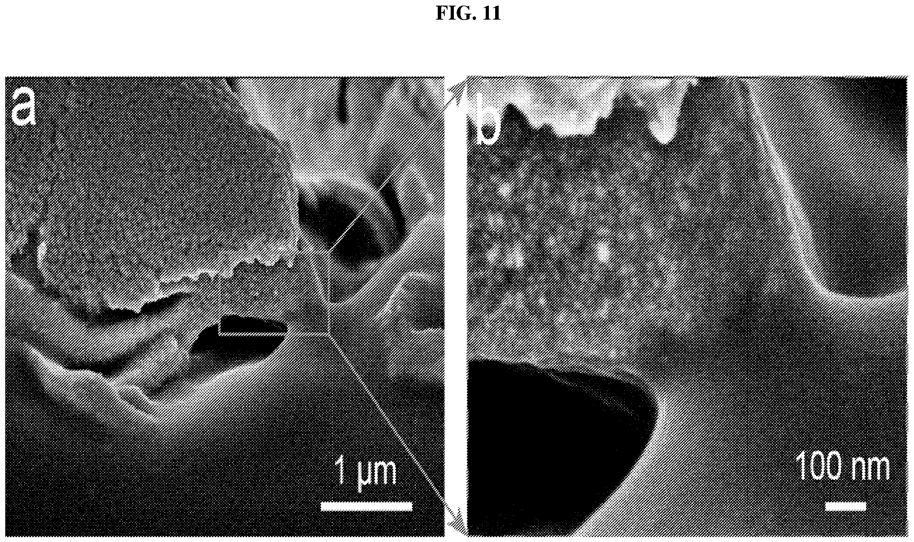

FIG. 11 shows (a) a scanning electron microscopy image (SEM) showing the mingling effect of the evaporated gold and the polydimethylsiloxane (PDMS) according to various embodiments, and (b) a magnified image of the boxed region indicated in (a).

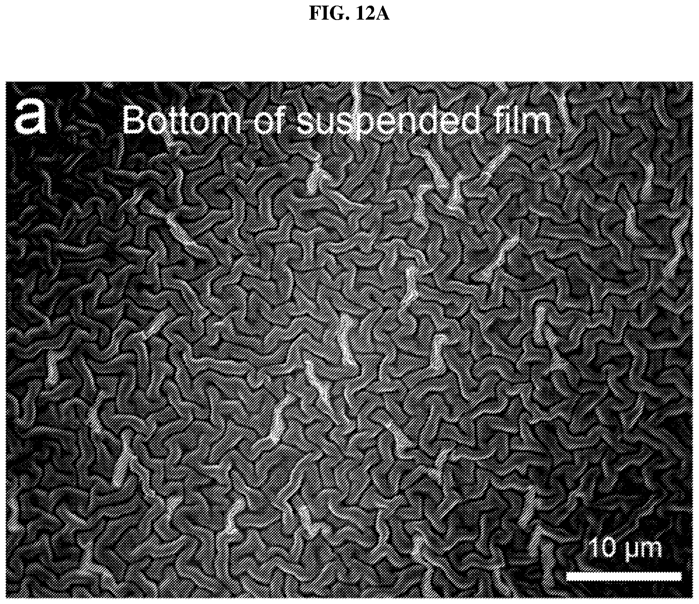

FIG. 12A is a scanning electron microscopy (SEM) image showing the surface morphology of the bottom of the suspended layers according to various embodiments.

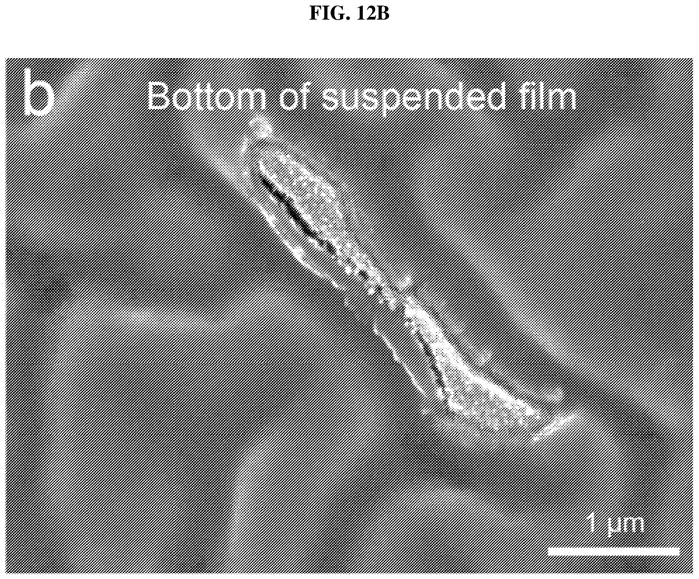

FIG. 12B is a magnified scanning electron microscopy (SEM) image of a region shown in FIG. 12A according to various embodiments.

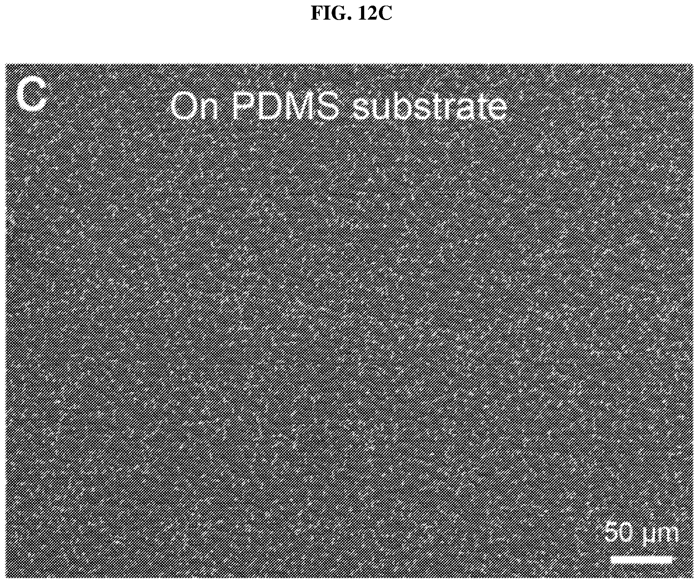

FIG. 12C is a scanning electron microscopy (SEM) image showing the surface morphology of the polydimethylsiloxane (PDMS) base layer according to various embodiments.

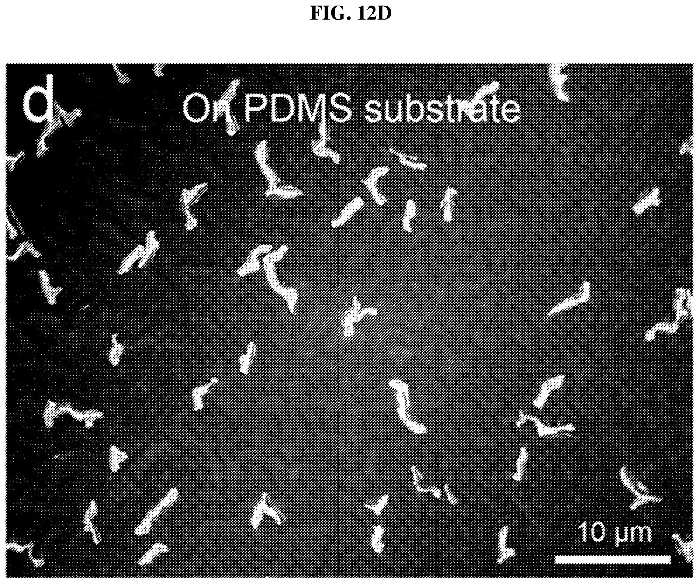

FIG. 12D is a magnified scanning electron microscopy (SEM) image of a region shown in FIG. 12C according to various embodiments.

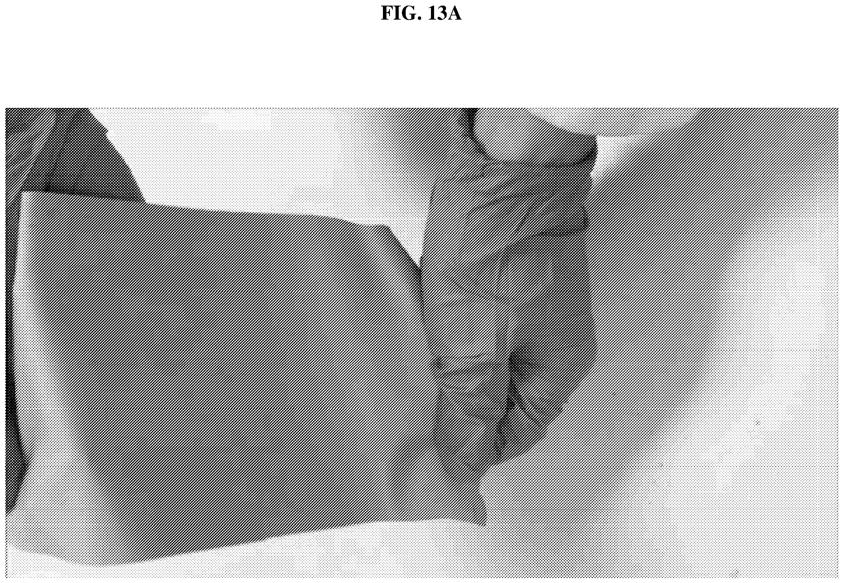

FIG. 13A is an image showing a large stretchable electrode according to various embodiments.

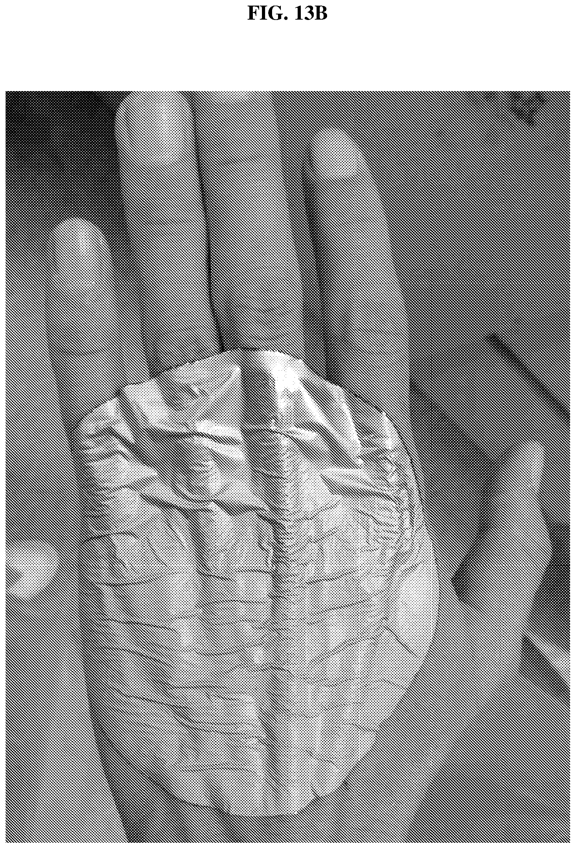

FIG. 13B is another image of a stretchable electrode according to various embodiments.

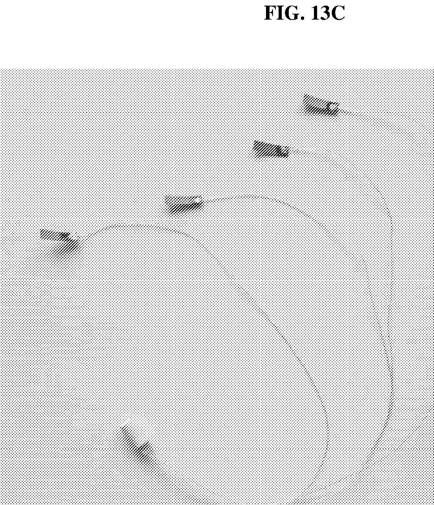

FIG. 13C is an image of fully encapsulated stretchable electrodes according to various embodiments.

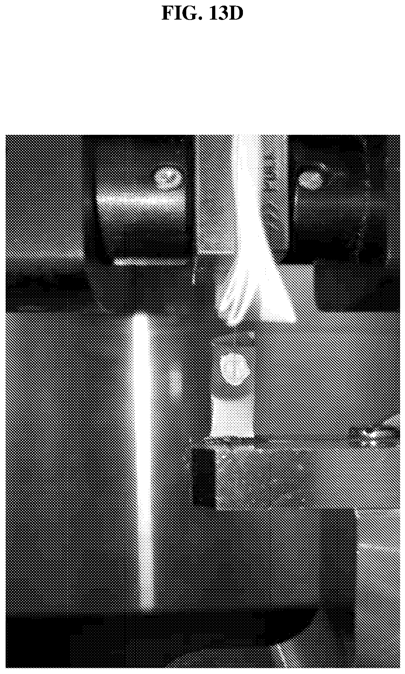

FIG. 13D is an image showing the stretchability of encapsulated stretchable electrodes according to various embodiments.

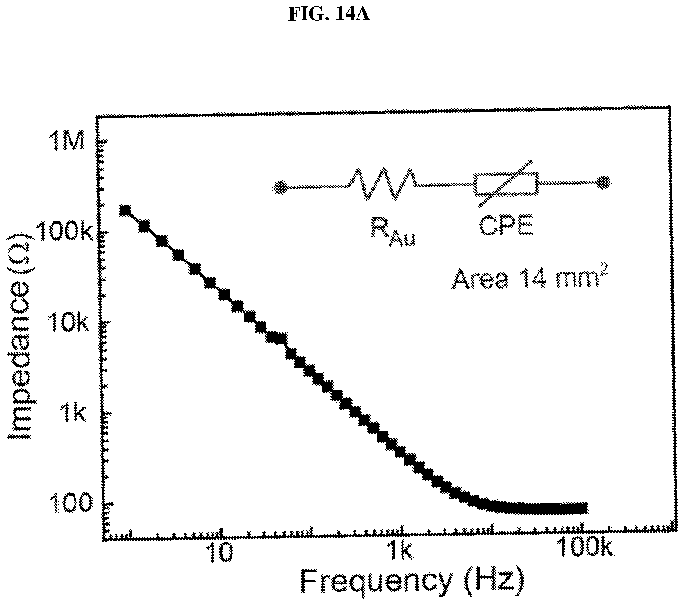

FIG. 14A shows a plot of impedance (in ohms or $\Omega$) as a function of frequency (in hertz or Hz) of a stretchable electrode according to various embodiments while the inset shows the fitting model.

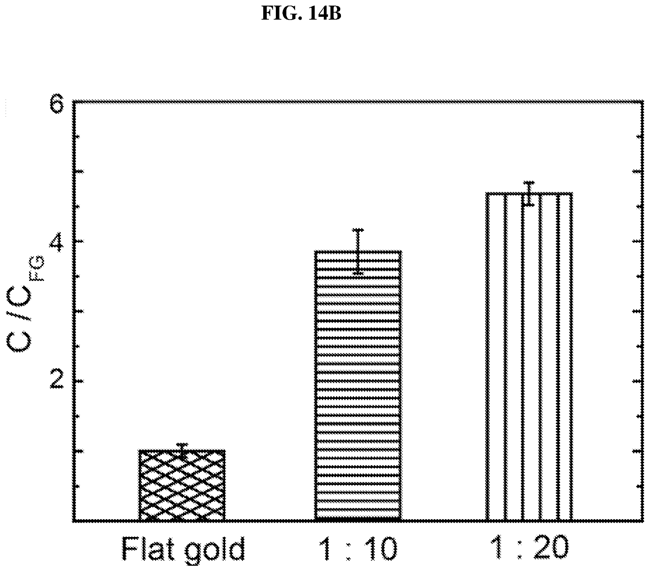

FIG. 14B is a plot of normalized capacitance comparing the capacitances of a flat gold film (normalized as 1), a first stretchable electrode (formed with a ratio of cross linker to monomer of 1 : 10) according to various embodiments, and a second stretchable electrode (formed with a ratio of cross linker to monomer of 1 : 20) according to various embodiments.

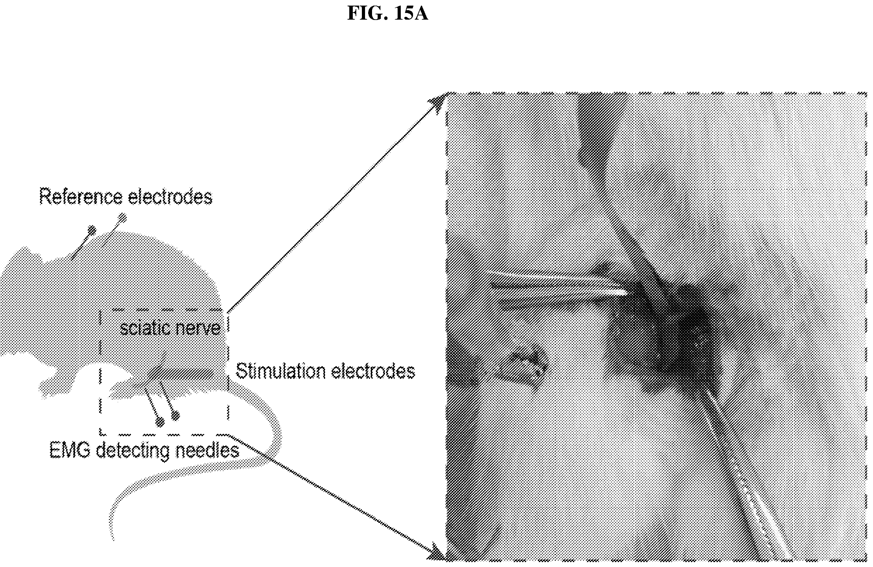

FIG. 15A shows (left) a schematic showing the implantation of the stretchable electrode according to various embodiments in a rat to stimulate the sciatic nerve, and (right) an image showing the implantation of the electrode according to various embodiments.

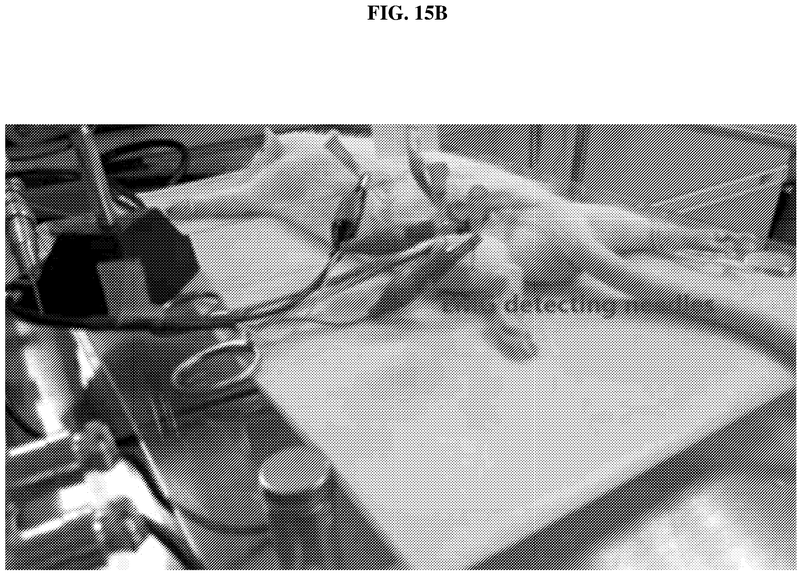

FIG. 15B is an image showing the recording of the electromyography (EMG) signal and the providing of the stimulation signal using the stretchable electrodes according to various embodiments.

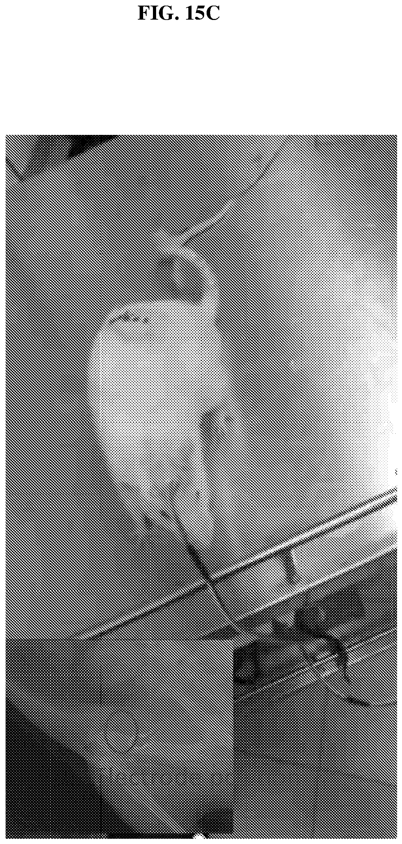

FIG. 15C is another image showing the rat implanted with the stretchable electrodes according to various embodiments, with the inset showing an X-ray image of the implanted electrode within the rat.

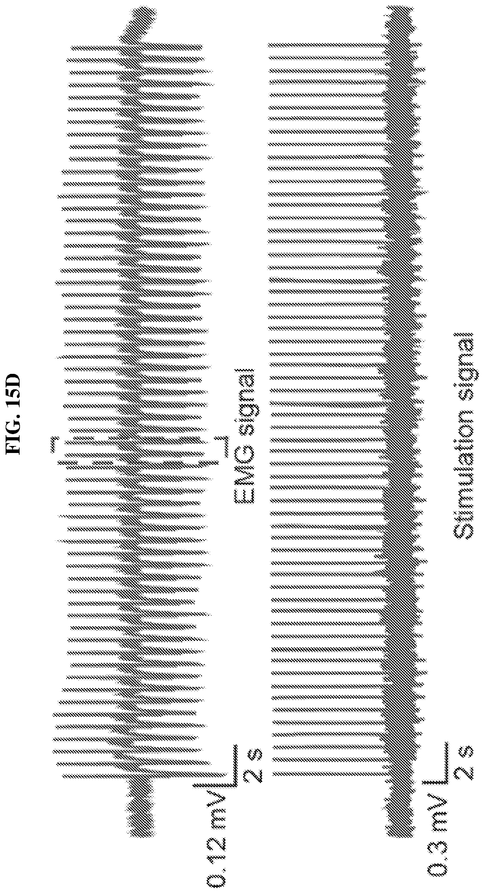

FIG. 15D is a plot of voltage (in millivolts or mV) as a function of time (in milliseconds or ms) showing the electromyography (EMG) signal detected and the stimulation signal applied to the electrode according to various embodiments.

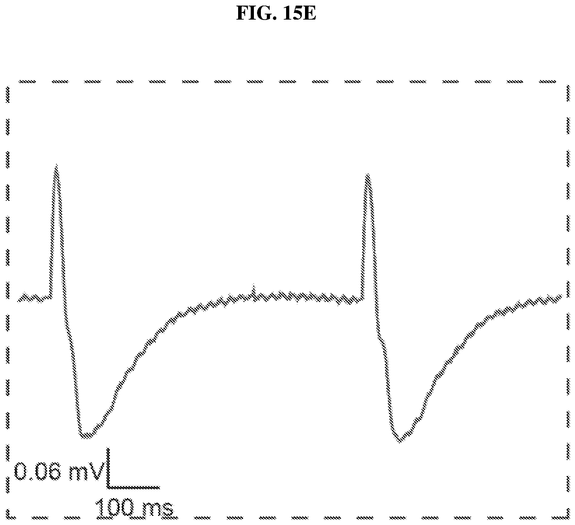

FIG. 15E shows a magnification of the electromyography (EMG) signal shown in FIG. 15D.

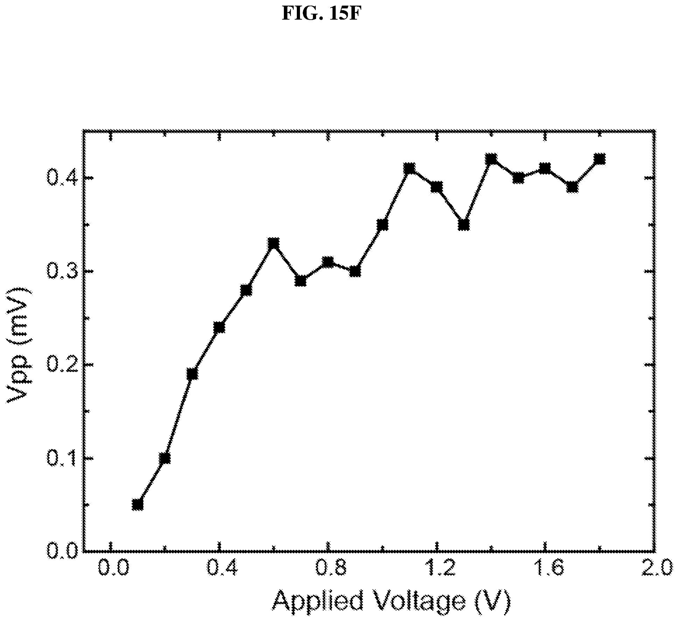

FIG. 15F is a plot of peak-to-peak value of the induced electromyography (EMG) signal as a function of the simulation voltage applied to the stretchable electrode according to various embodiments.

## DETAILED DESCRIPTION

The following detailed description refers to the accompanying drawings that show, by way of illustration, specific details and embodiments in which the invention may be practiced. These embodiments are described in sufficient detail to enable those skilled in the art to practice the invention. Other embodiments may be utilized and structural, and logical changes may be made without departing from the scope of the invention. The various embodiments are not necessarily mutually exclusive, as some embodiments can be combined with one or more other embodiments to form new embodiments.

Embodiments described in the context of one of the methods or stretchable electrodes are analogously valid for the other methods or stretchable electrodes. Similarly, embodiments described in the context of a method are analogously valid for a stretchable electrode, and vice versa.

Features that are described in the context of an embodiment may correspondingly be applicable to the same or similar features in the other embodiments. Features that are described in the context of an embodiment may correspondingly be applicable to the other embodiments, even if not explicitly described in these other embodiments. Furthermore, additions and/or combinations and/or alternatives as described for a feature in the context of an embodiment may correspondingly be applicable to the same or similar feature in the other embodiments.

The word "over" used with regards to a deposited material formed "over" a side or surface, may be used herein to mean that the deposited material may be formed "directly on", e.g. in direct contact with, the implied side or surface. The word "over" used with regards to a deposited material formed "over" a side or surface, may also be used herein to mean that the deposited material may be formed "indirectly on" the implied side or surface with one or more additional layers being arranged between the implied side or surface and the deposited material. In other words, a first layer "over" a second layer may refer to the first layer directly on the second layer, or that the first layer and the second layer are separated by one or more intervening layers.

The stretchable electrode as described herein may be operable in various orientations, and thus it should be understood that the terms "top", "bottom", etc., when used in the following description are used for convenience and to aid understanding of relative positions or directions, and not intended to limit the orientation of the stretchable electrode.

In the context of various embodiments, the articles "a", "an" and "the" as used with regard to a feature or element include a reference to one or more of the features or elements.

In the context of various embodiments, the term "about" or "approximately" as applied to a numeric value encompasses the exact value and a reasonable variance.

As used herein, the term "and/or" includes any and all combinations of one or more of the associated listed items.

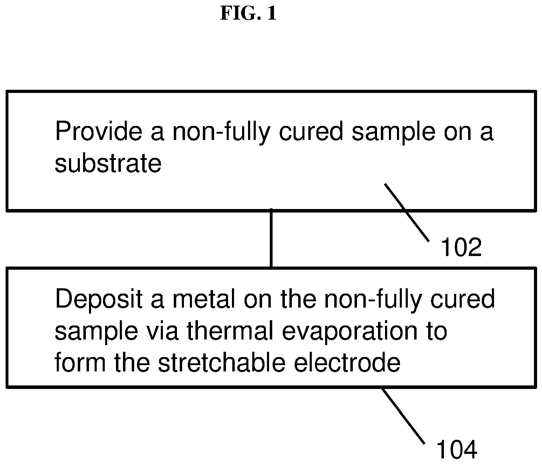

FIG. 1 is a schematic illustrating a method of forming a stretchable electrode according to various embodiments. The method may include, in 102, providing a non-fully cured sample on a substrate. The method may also include, in 104, depositing a metal on or over the non-fully cured sample via thermal evaporation to form the stretchable electrode. The non-fully cured sample may be uncured or is partially cured for a duration of less than 30 minutes.

In other words, the method may include introducing a suitable metal to any one selected from a group consisting of an uncured sample and a partially cured sample. The metal may be introduced using thermal evaporation.

In various embodiments, the uncured sample may include a monomer and a cross-linker. The mixture of the monomer and a cross-linker may be provided onto the substrate. There may not yet be any polymerization and/or cross-linking occurring in the mixture at the time in which to mixture is provided onto the substrate. In various embodiments, the uncured sample may additionally include an oligomer. In various embodiments, the uncured sample may include an oligomer and a cross-linker.

In various embodiments, the partially cured sample may include a plurality of polymeric chains joined to another via one or more cross-links. There may be some polymerization in the partially cured sample with some cross-links.

However, the partially cured sample may not yet be fully polymerized. The cross-linking in the polymeric chains may be incomplete. In other words, not all the cross-links in the partially cured sample have been formed. The partially cured sample is formed (from a mixture of a monomer and a cross-linker) by heating.

The heating (during the pre-curing stage) is carried out at a temperature of less than 70 °C, e.g. at about 60 °C. In various embodiments, the non-fully cured sample may be partially cured for a duration of less than 26 minutes, less than 25 minutes, less than 20 minutes, less than 15 minutes, or about 15 minutes. In various embodiments, the pre-curing and the deposition may be carried out in different apparatuses. The pre-curing may be carried out in an oven, while the evaporation may be carried out in a thermal evaporation machine. The conditions for pre-curing may also be different from the conditions for deposition.

In various embodiments, the partially cured sample may also include a portion of the monomer and a portion of the cross-linker which have not yet undergone reaction.

In various embodiments, the substrate may be a rigid substrate or backing such as a glass slide.

The method may further include providing a metal source comprising the metal. The metal source may face the non-fully cured sample. For example, the non-fully cured sample may face downwards, while the metal source may face upwards. Evaporation may generally involve applying heat to vaporise a source material, such as the metal source. The source material may be arranged within a chamber. Evaporation may include thermal evaporation or electron-beam evaporation. Thermal evaporation may include heating the metal source or metal via a current applied. The current may be applied to a heating element such as an electric filament, which may then be heated up. The heat generated may then be transferred to the metal source or metal. Electron-beam evaporation may involve heating the metal source or metal using an electron beam.

The deposition of the metal on the non-fully cured sample may be carried out in a vacuum. In other words, the chamber may include a vacuum. The vacuum may allow vapor particles to travel directly to the target object, i.e., the sample, where they condense back to a solid state.

The vacuum may be less than $10^{-5}$ Torr, less than $5.0 \times 10^{-6}$ Torr, less than $2.0 \times 10^{-6}$ Torr, e.g. at about $1.0 \times 10^{-6}$ Torr.

The metal may be any one selected from a group consisting of gold, palladium, aluminium and copper.

In various embodiments, the method may also include depositing one or more further metals on or over the non-fully cured sample via thermal evaporation to form the stretchable electrode. For instance, chromium may first be deposited over the non-fully cured sample, followed by gold. The method may further include providing one or more further metal sources.

The non-fully cured sample may be fully cured during deposition of the metal. The non-fully cured sample may be fully cured due to the thermal energy provided to the non-fully cured sample during the deposition process. For instance, the non-fully cured sample may receive thermal radiation from the metal source(s), and may also receive thermal energy by conduction when the hot metal particles are deposited onto the non-fully cured sample.

The fully cured sample may be a sample with complete cross-links. In other words, all the cross-links in the fully cured sample may be formed. In a fully cured sample, the polymer chains are held together by the cross-links and may not slide easily pass one another. On the other hand, in a non-fully cured sample, the polymer chains may slide pass one another easily due to the relatively fewer cross-linking sites.

The polymer layer and the metal film formed on the polymer layer may protrude or enter into each other. The non-fully cured sample may have an interface region in which the polymer layer, i.e. the wrinkled layer, and the metal film intersperse with each other. As more metal is deposited on the non-fully cured sample to form the metal film (during the fabrication process), during the polymerization process of the polymer, the wrinkled morphology may form due to the mechanical mismatching of the gold film and polymer. The sample may include a stripped hollowed layer formed by the pulling down of parts of the polymer layer under gravity.

The stretchable electrode includes a base layer. The stretchable electrode also includes the wrinkled layer. The stretchable electrode further includes a plurality of pillars, e.g. micropillars, extending from the base layer to the wrinkled layer. The base layer, the wrinkled layer, and the plurality of pillars include or are made of a polymer. The stretchable electrode additionally includes a metal film on the wrinkled layer. The metal film includes the metal. The wrinkled layer may be spaced apart from the base layer via a spacing or cavity, and may be supported by the plurality of pillars extending through the spacing or cavity from the base layer. The wrinkled layer may have an irregular and rough surface. The stretchable electrode may be described as having a karst caves-like structure. The base layer may be on or in contact with the substrate.

The polymer is polydimethylsiloxane (PDMS).

Various embodiments may relate to a stretchable electrode formed by any method as described herein.

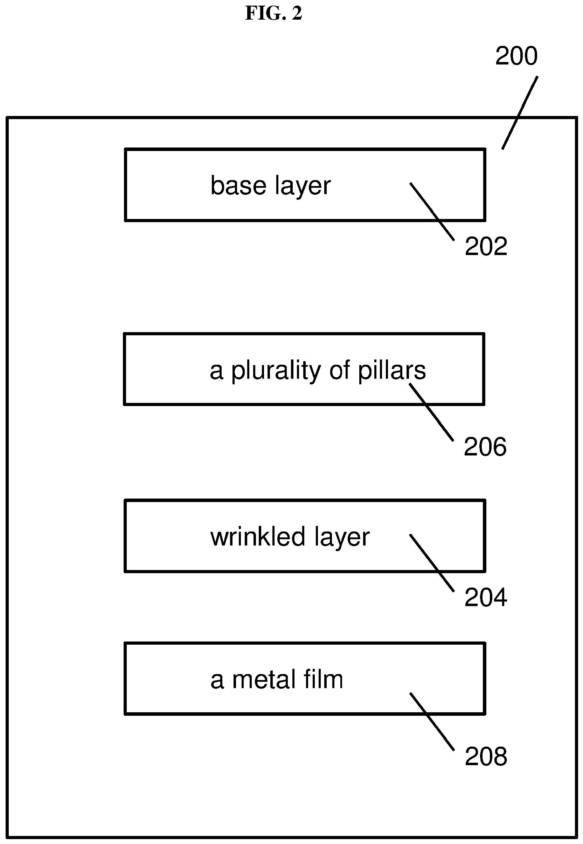

FIG. 2 is a general illustration of a stretchable electrode 200 according to various embodiments. The stretchable electrode 200 includes a base layer 202. The stretchable electrode 200 also includes a wrinkled layer 204. The stretchable electrode 200 further includes a plurality of pillars 206 extending from the base layer 202 to the wrinkled layer 204. The stretchable electrode 200 additionally includes a metal film 208 on the wrinkled layer 204. The base layer 202, the wrinkled layer 204, and the plurality of pillars 206 include a polymer.

In other words, the electrode 200 includes a base layer 202 and a wrinkled layer 204 held above the base layer 202 by a plurality of pillars. The electrode 202 further includes a metal film 208 in contact with the wrinkled layer 204.

The wrinkled layer 204 may be spaced apart from the base layer 202 via a spacing or cavity, and may be supported by the plurality of pillars 206 extending through the spacing or cavity from the base layer 202. The wrinkled layer 204 may have an irregular and rough surface. The stretchable electrode 200 may be described as having a karst caves-like structure.

An adhesion strength of the metal film 208 to the wrinkled layer may be higher than 1 MPa.

The stretchable electrode 200 may have a stretchability exceeding 130%.

Various embodiments may relate to a method of utilizing dynamic interface mingling to fabricate high performance stretchable electrodes, which may also be referred to as stretchable conductors.

In contrast with traditional fabrication methods, in various embodiments, uncured or partially cured PDMS is used to accept metal nanoparticles, e.g. gold nanoparticles, generated by thermal evaporation. The evaporated metal may mingle with the polymer dynamically at the interface.

The three physical-chemical processes including (1) mingling effect, (2) polymerization due to the heat radiation emitted by the metal source used in thermal evaporation and due to the energy carried by the evaporated gold, and (3) the stripping of a metal-polymer (e.g. gold-PDMS) ultrathin layer under gravity may happen at the same time.

A large area self-stripped karst caves-like structure with wrinkled surface may be fabricated. The structure may redistribute the strain from concentration mode present in flat films to random mode, hence achieving high stretchability.

The mingling effect may enhance the adhesion significantly. The wrinkled structure may increase the surface area of the electrode.

The stretchable electrode or conductor may be used to monitor mechanical deformation and electrophysiology signals. The stretchable electrode or conductor may also act as an implantable nerve stimulator to facilitate neural regeneration/rehabilitation.

Various embodiments may lead to a solid improvement towards various practical applications of stretchable electrodes, which may be a composite film including a metal and a polymer. Various embodiments may also relate to a stretchable composite film involving other materials.

Experimental Details

Fabrication of Stretchable Electrodes or Conductors According to an Embodiment

Glass slides without any pre-treatment were used as the rigid backing for uncured PDMS (184, Dow Corning) with different ratio of cross linker to monomer e.g. 1 : 5, 1 : 10, and 1 : 20. The uncured PDMS was subjected to different pre-curing times of 15, 20, 25 minutes respectively in a 60 °C oven. The samples may be fully cured by putting the samples in the 60 °C oven for 6 hours.

Some samples were placed into a thermal evaporation machine with the partially cured PDMS samples facing down towards a gold source or a chromium source. The vacuum was then reduced to $1.0 \times 10^{-6}$ Torr, and the chromium source or gold source was heated up. The chromium and gold were evaporated in sequence at the rate of 0.2 Å s$^{-1}$ and 0.5 Å s$^{-1}$ respectively to form the stretchable electrode including PDMS, chromium and gold. The chromium may act as an adhesion layer between gold and PDMS. The stretchable electrode formed may include a polymer (i.e. PDMS) wrinkled layer held above a polymer (i.e. PDMS) base layer by a plurality of polymer (i.e. PDMS) pillars. The stretchable electrode may further include a chromium layer on the wrinkled layer, and a gold layer on the wrinkled chromium layer.

In some other samples, only gold was evaporated to form a stretchable electrode including PDMS and gold. The stretchable electrode formed includes a PDMS wrinkled layer held above a PDMS base layer by a plurality of polymer (i.e. PDMS) pillars. The stretchable electrode may further include a gold layer on the wrinkled layer.

Preparation of Conventional Stretchable Films and Flat Non-stretchable Gold Films

Conventional stretchable films and flat non-stretchable gold films were also prepared as control samples. Conventional stretchable gold films were fabricated by controlling the growth mode of gold films on fully cured PDMS via forming islands of gold. The final gold films had randomly-distributed nanodefects when the gold islands coalesced or connected with one another. These nanodefects would induce randomly-distributed micro cracks when under tensile strain, resulting in the gold film having a network structure. On the other hand, flat non-stretchable films did not possess initial nanodefects and were flat dense films. Under tensile strain, the non-stretchable films would develop large throughout cracks without stretchability.

The conventional stretchable films were fabricated by depositing gold onto fully cured PDMS without any pre-treatment. The evaporation rate was 0.3 angstrom per second. The vacuum used was $3 \times 10^{-6}$ Torr. The flat non-stretchable gold films were fabricated by using the same deposition condition on oxygen plasma treated fully cured PDMS.

The conventional crack-based stretchable gold film had an appearance of brown color due to the thin film thickness imparted by the special morphology of initial nano cracks. The thickness of the non-stretchable film was around 80 nm. The upper limit of the thickness for the stretchable film was around 100 nm.

Peel Test Of Tensile Adhesion Strength

The tensile adhesion strength was obtained following a conventional test process. The cylindrical wood sticks were adhered to samples by epoxy resin. A MTS C42 with 50 N load cell was employed to test the adhesion force when the stick was pulled up by the Bionix vice grips. The synchronous measurements of resistance and tensile strain were also performed.

Animal Surgery And Neural Stimulation

Adult male Sprague-Dawley rats (150 - 200g) were obtained from Guangdong Medical Experimental Animal Center (Guangdong, China). All procedures were carried out under the guidelines of the Institutional Animal Care and Use Committee of Shenzhen Institutes of Advanced Technology, Chinese Academy of Sciences. The anesthesia and surgery followed standard procedures. The electrode was carefully implanted and entwined around the sciatic nerve. The stimulation and EMG were recorded using Medlab biological signal collecting and handling system.

Previously, PDMS is used after being fully cured. A typical process may involve a fully cured PDMS undergoing a certain pre-treatment, such as oxygen plasma cleaning, followed by deposition of a metal film or a semiconductor film to form a conductive film or electrode.

Examples

In contrast, various embodiments may involve deposition of metal on an uncured or a partially cured sample. The evaporated metal may mingle with the monomer, oligomer or partially cured polymer in a series of dynamic physical-chemical processes. The metal and monomer, oligomer/polymer may protrude or enter into each other along the interface, resulting in high adhesion of the metal film to the fully cured polymer formed after the deposition process.

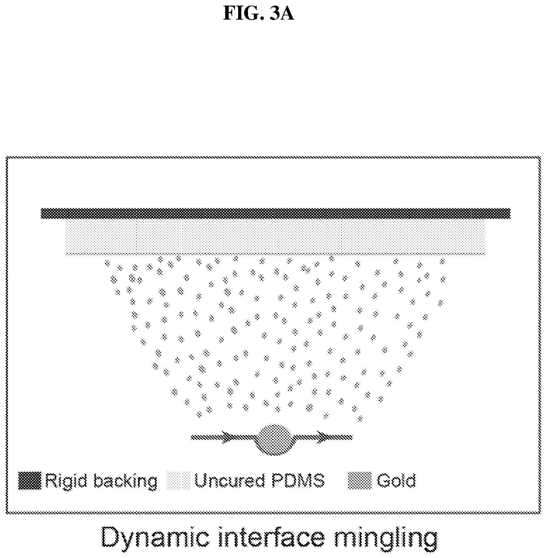

FIG. 3A shows (top) a plurality of gold clusters/nanoparticles being evaporated onto non-fully cured polydimethylsiloxane (PDMS) on a rigid backing, and (bottom) evaporated gold clusters/nanoparticles being received by the non-fully cured polydimethylsiloxane (PDMS) according to various embodiments.

The PDMS and gold may mingle together and protrude or enter into each other along the gold-PDMS interface, after the hot, fast speed gold nanoparticles strike onto the soft uncured or partially cured PDMS. During the mingling process, the uncured or partially cured PDMS process may undergo polymerization and cross-linking. The polymerization and cross-linking may be enhanced due to the thermal radiation of the gold source (which may have a temperature of up to 1800 K), and due to heat transfer from the hot, newly deposited gold.

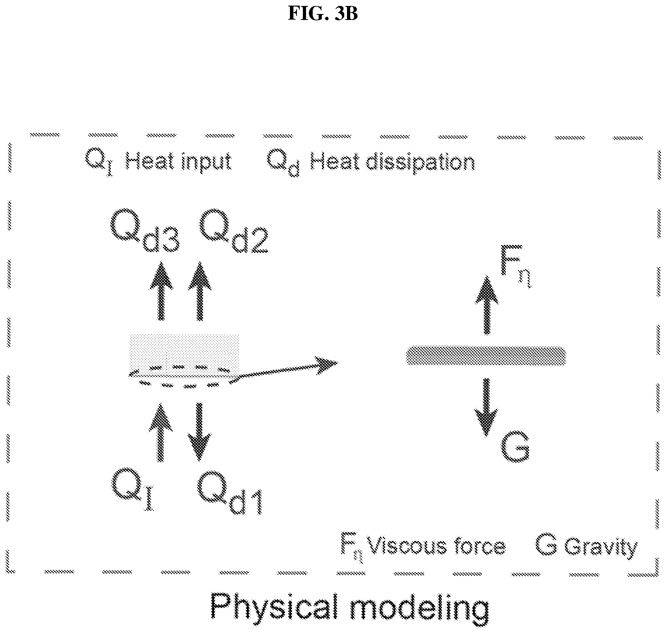

FIG. 3B is a schematic illustrating physical models of heat transfer and viscous flow occurring during the dynamic process of gold deposition onto non-fully cured polydimethylsiloxane (PDMS) according to various embodiments.

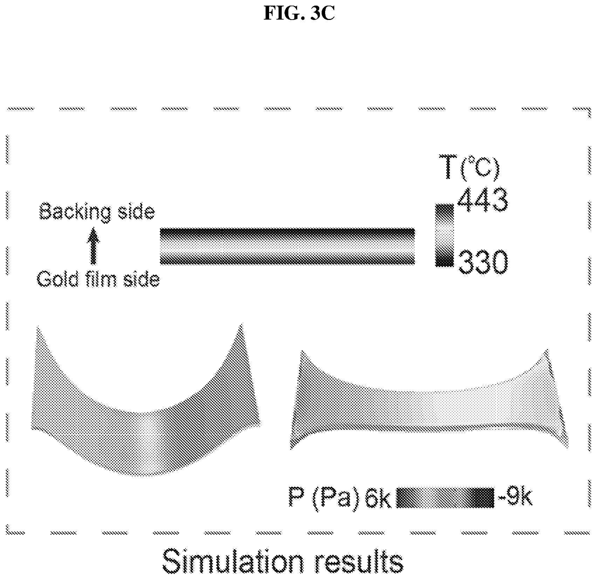

FIG. 3C is a schematic showing simulation results of corresponding temperature distribution and pressure distribution during viscous flow (low viscous flow on the left and high viscous flow on the right) when gold is deposited onto non-fully cured polydimethylsiloxane (PDMS) according to various embodiments.

As shown in FIG. 3B, the quasi-steady-state heat transfer model may include the processes of heat input from the thermal radiation and the hot deposited gold, $Q_I$, heat dissipation from the thermal radiation at both sides of the film, $Q_{d1}$ and $Q_{d2}$, and conduction at the backing, $Q_{d3}$ (heat convection can be omitted). Under the quasi-steady state model,

$$Q_I \approx Q_{d1} + Q_{d2} + Q_{d3} \tag{1}$$

The temperature distribution in FIG. 3C may be roughly obtained based on Equation (1).

Due to the low thermal conductivity of PDMS and the relatively slow thermal dissipation rate, a temperature gradient may exist within the structure. As shown in FIG. 3C, the temperature at the backing side of the PDMS structure may be about 330 °C, while the temperature at the gold film side may be about 443 °C. The maximum temperature difference may be ~ 120 °C.

The high temperature at the gold film side may significantly speed up the polymerization at the gold film side. Based on theoretical simulation, when the thermal diffusion is not in steady state, the temperature of the gold film side near to the gold source may even reach a temperature such that the temperature difference between the temperature at the gold film side and the temperature at the backing side exceeds 120 °C.

A mechanical process may also occur simultaneously during the gold deposition process, which is illustrated in FIG. 3B. The density of gold (19,300 kg m$^{-3}$) is much higher than that of PDMS (0.97 kg m$^{-3}$). Accordingly, with the increase in deposited gold at the interface, the gold may pull down a thin layer of PDMS. At the same time, the partially cured PDMS may become highly-viscous rapidly as the temperature goes up.

Eventually, the self-weight G of the gold-PDMS interface layers may balance the viscosity force of PDMS, $F_\eta$, and the viscous PDMS interface layer may not move further downwards once the viscosity is large enough. The pressure distributions in PDMS layers of different viscosities are illustrated in FIG. 3C. The shape of the structure may be rapidly frozen as a result of the increase in temperature.

Since the thermal evaporation rate is slow (usually ~ 0.5 Å s$^{-1}$) and takes around 33 minutes to deposit a 100 nm-thick film, the whole elastic PDMS sample may be fully polymerized and cured during the process. Thus, the resultant structure formed may include a suspended layered composite including a wrinkled PDMS layer and a wrinkled gold layer in contact with the wrinkled PDMS layer. The wrinkled PDMS layer and the wrinkled gold layer may be due to the mingling effect at the interface, and may be supported by pulled-out micro pillars during above described dynamic processes.

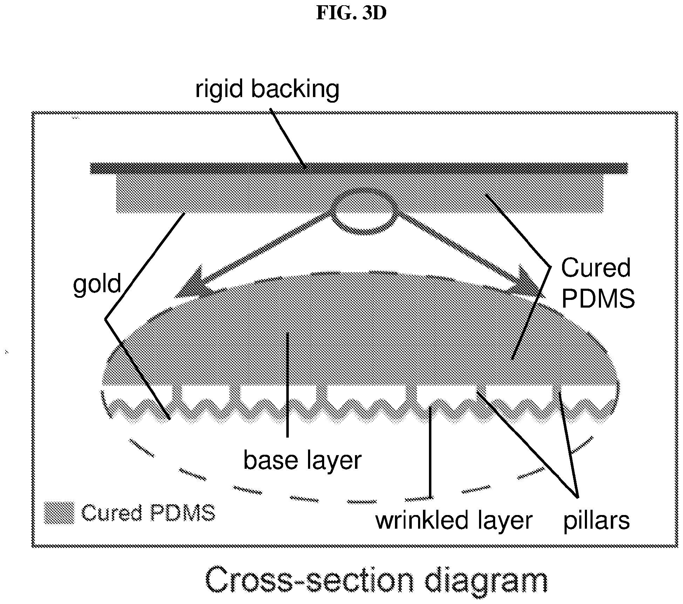

FIG. 3D is a schematic showing a resulting structure of the stretchable electrode according to various embodiments. The structure may include a stripped hollowed layer formed by the pulling down of the wrinkled polymer layer by the metal film. The structure shown in FIG. 3D may have high stretchability, good stability, high adhesion and large surface area. The method described herein may thus offer an approach to form a stretchable electrode with the abovementioned advantages.

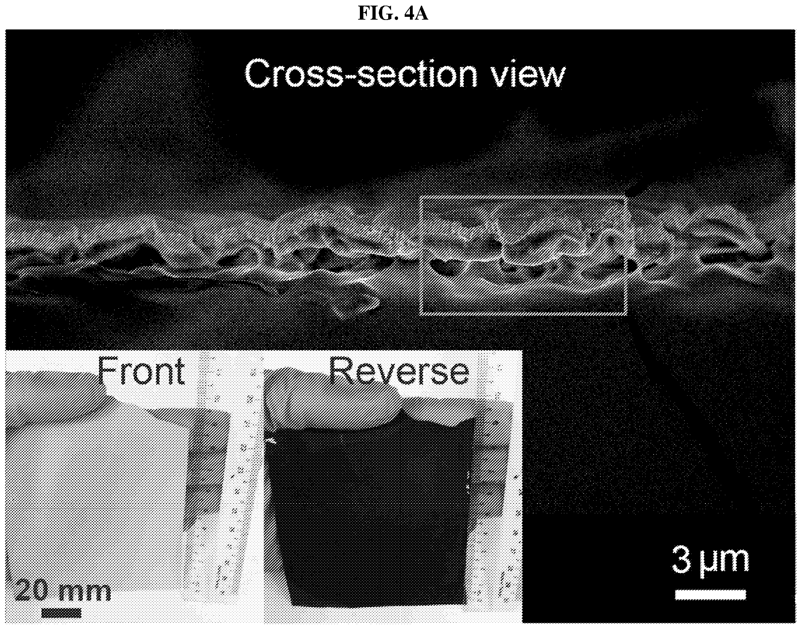

FIG. 4A shows a cross-sectional scanning electron microscopy (SEM) image of the self-stripping double-layer structure according to various embodiments, with the insets showing optical images of the front view and back view of the sample according to various embodiments. The insets indicate that samples with large area may be formed. The back of the sample may be black resulting from the absorption effect of the gold nanoparticles mingled with PDMS. Considering the relatively simple fabrication process and large scale yield, various embodiments may be one of the best candidates for stretchable and flexible electrodes.

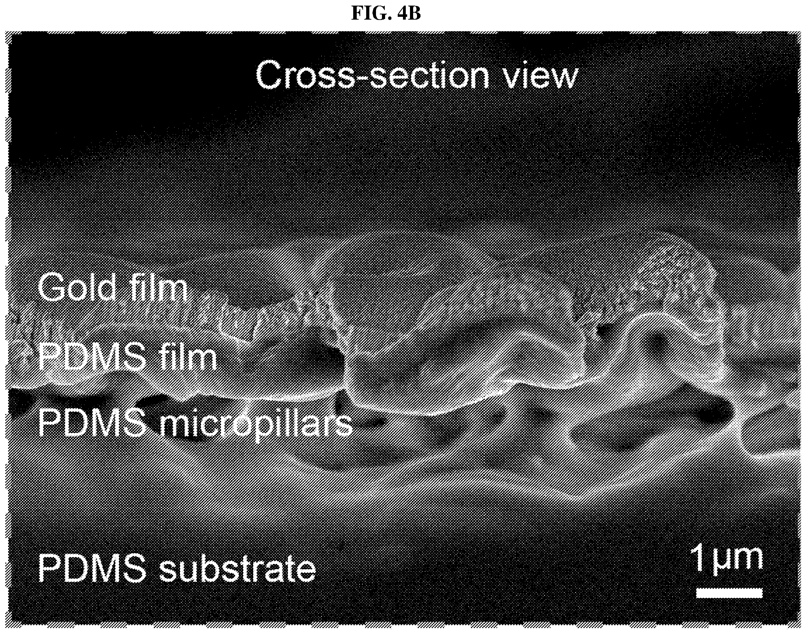

FIG. 4B shows a magnified cross-sectional scanning electron microscopy (SEM) image of the self-stripping double-layer structure according to various embodiments shown in FIG. 4A. FIG. 4B corresponds to the section in FIG. 4A indicated by the rectangular box.

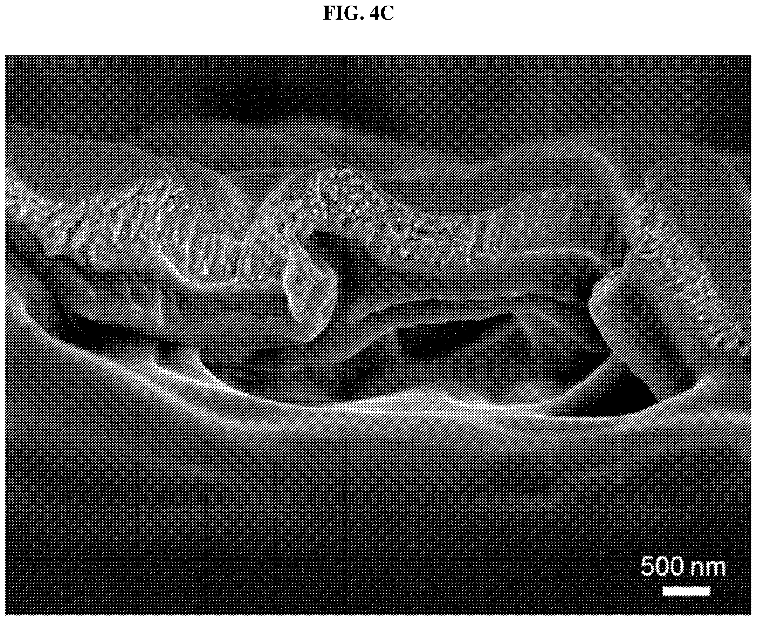

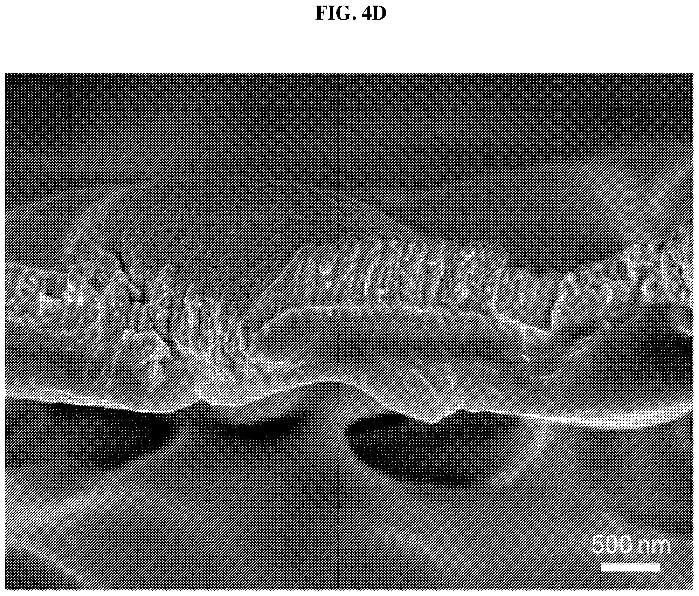

FIG. 4C shows another magnified cross-sectional scanning electron microscopy (SEM) image of the self-stripping double-layer structure according to various embodiments shown in FIG. 4A. FIG. 4D shows yet another magnified cross-sectional scanning electron microscopy (SEM) image of the self-stripping double-layer structure according to various embodiments shown in FIG. 4A.

From the SEM images shown in FIGS. 4A-D, a karst caves-like structure may be observed. The structure may include a suspended PDMS layer with a gold layer in contact with the PDMS layer. The interface region between the PDMS layer and the gold layer may be a transition region clearly showing the mingling effect. The suspended layers may be linked to the PDMS base layer (also referred to as PDMS substrate layer) by micro pillars.

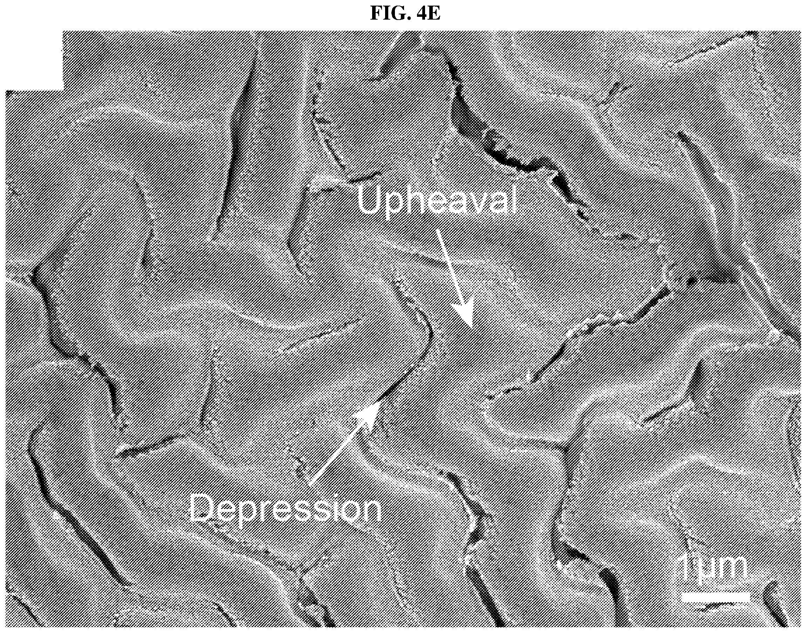

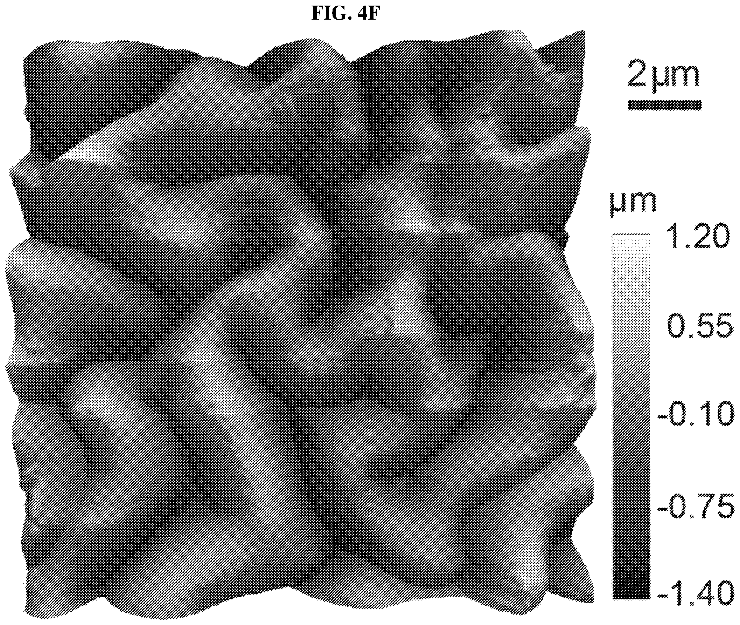

FIG. 4E shows a scanning electron microscopy (SEM) image of the gold film having the wrinkled structure according to various embodiments. The wrinkled structure may include upheavals and depressions. FIG. 4F shows an atomic force microscopy (AFM) image of the gold film having the wrinkled structure according to various embodiments. The surface morphology of the gold film shown in FIG. 4E and FIG. 4F and underlying wrinkled PDMS layer may be induced by the modulus mismatch between gold and PDMS during dynamic polymerization.

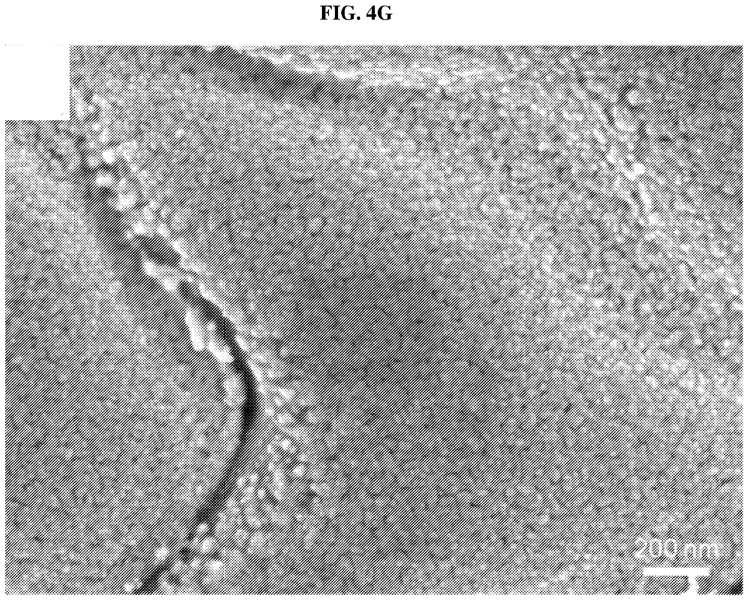

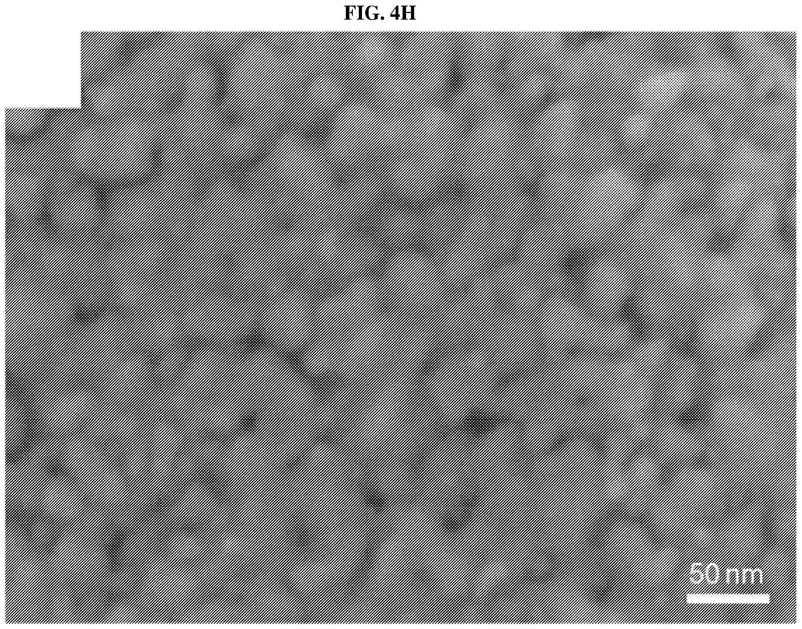

FIG. 4G is an image showing the detailed morphology of the gold film according to various embodiments. FIG. 4H is another image showing the detailed morphology of the gold film according to various embodiments. FIGS. 4G and 4H show that the wrinkled gold film may include gold nanoparticles having a grain size of about 20 nm.

The effects of weight ratio of the cross linker to the PDMS monomer as well as the pre-cured time on the surface morphology and subsequent performance are also studied. Different wrinkled surfaces may be obtained under different fabrication conditions.

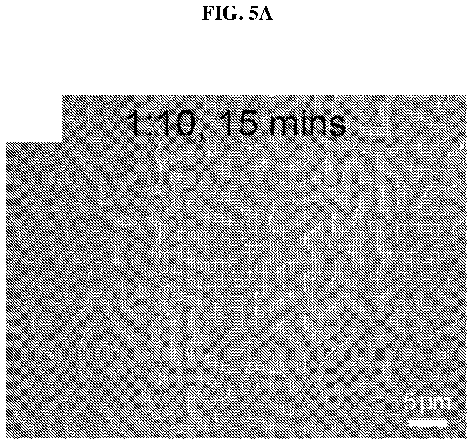

FIG. 5A shows a planar surface electron microscopy (SEM) image formed from a sample pre-cured for a duration of 15 minutes and having a ratio of cross linker to monomer of 1 : 10 according to various embodiments.

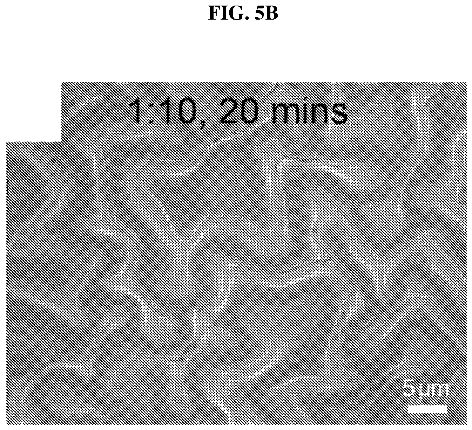

FIG. 5B shows a planar surface electron microscopy (SEM) image formed from a sample pre-cured for a duration of 20 minutes and having a ratio of cross linker to monomer of 1 : 10 according to various embodiments.

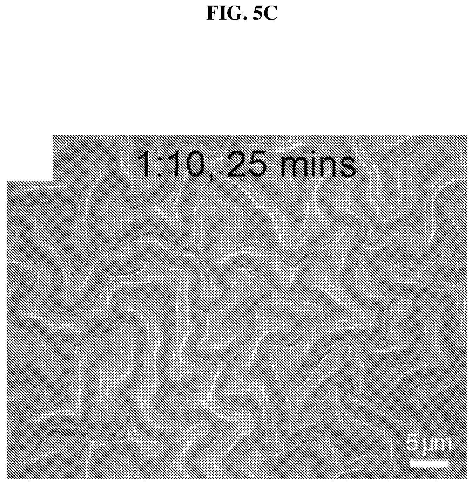

FIG. 5C shows a planar surface electron microscopy (SEM) image formed from a sample pre-cured for a duration of 25 minutes and having a ratio of cross linker to monomer of 1 : 10 according to various embodiments.

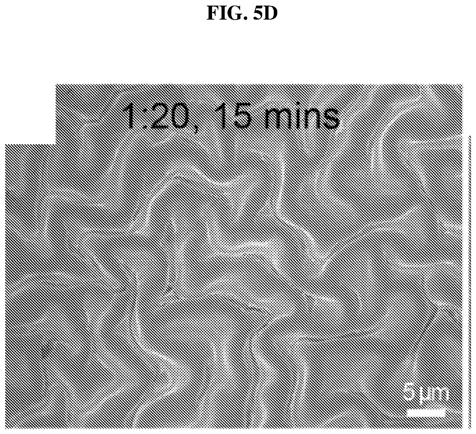

FIG. 5D shows a planar surface electron microscopy (SEM) image formed from a sample pre-cured for a duration of 15 minutes and having a ratio of cross linker to monomer of 1 : 20 according to various embodiments.

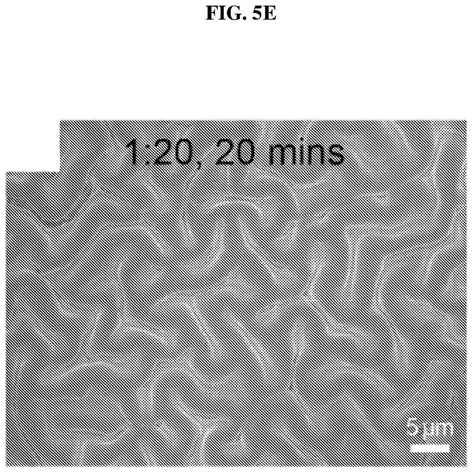

FIG. 5E shows a planar surface electron microscopy (SEM) image formed from a sample pre-cured for a duration of 20 minutes and having a ratio of cross linker to monomer of 1 : 20 according to various embodiments.

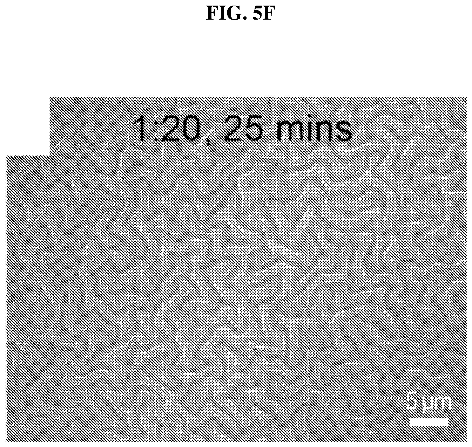

FIG. 5F shows a planar surface electron microscopy (SEM) image formed from a sample pre-cured for a duration of 25 minutes and having a ratio of cross linker to monomer of 1 : 20 according to various embodiments.

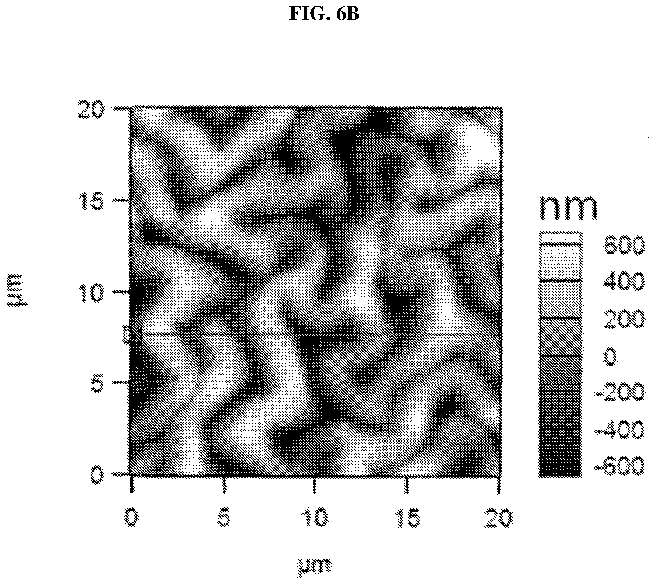

FIGS. 6A-F compare the wrinkles present at a surface of a wrinkled composite film including a gold layer and a suspended PDMS layer. FIG. 6A shows an atomic force microscopy (AFM) image of a wrinkled film according to various embodiments. FIG. 6B shows another atomic force microscopy (AFM) image of the wrinkled film shown in FIG. 6A according to various embodiments. FIG. 6C is a plot of amplitude of the wrinkles in nanometers or nm) as a function of distance along the surface (in micrometers or $\mu$m) of the film shown in FIGS. 6A-B according to various embodiments. The thickness of the film illustrated in FIGS. 6A-C is about 300 nm, and may be formed with a ratio of cross linker to monomer of 1 : 10 and pre-cured for a duration of 25 minutes before deposition.

FIG. 6D shows an atomic force microscopy (AFM) image of another wrinkled film according to various embodiments. FIG. 6E is a plot of amplitude of the wrinkles in nanometers or nm) as a function of distance along the surface (in micrometers or $\mu$m) of the film shown in FIG. 6D according to various embodiments. The thickness of the film illustrated in FIGS. 6D-E is about 300 nm, and is formed with a ratio of cross linker to monomer of 1 : 20 and pre-cured for a duration of 25 minutes before deposition.

FIG. 6F is a plot of maximum amplitude (in micrometers or $\mu$m) comparing a first film formed with a ratio of cross linker to monomer of 1 : 10 and pre-cured for a duration of 25 minutes before deposition, and a second film formed with a ratio of cross linker to monomer of 1 : 20 and pre-cured for a duration of 25 minutes before deposition according to various embodiments.

FIGS. 6A-F show that the wrinkles may become denser and the amplitude of the wrinkles may decrease as the monomer increases relative to the cross-linker, when the pre-cured time remains the same.

The wrinkles formed on the structures according to various embodiments may be different from that fabricated by the normal pre-stretch or pre-biaxial strain method. The normal pre-stretch or pre-biaxial strain method may be able to support a relatively thinner wrinkled metal film on top.

In order to get a similar pattern with the normal pre-stretch method or pre-biaxial strain method, a two-dimensional (2D) pre-strain may be applied inside the elastic substrate, and a metal layer may be deposited on the strained substrate. FIG. 7A shows a schematic illustrating the pre-biaxial strain method. FIG. 7B shows a scanning electron microscopy (SEM) image of the film prepared by the pre-biaxial strain method.

As shown in FIG. 7B. a two dimensional (2D) slightly wrinkled pattern may appear due to the mismatch of the modulus when the strain is released. However, the amplitude of these wrinkles may be highly dependent on the adhesion and the thickness of the metal layer. If the thickness is too large (like ~ 300 nm), it is hard to form a regular dense wrinkled structure according to the buckling theory.

FIG. 7C shows a scanning electron microscopy (SEM) image of the film prepared by the method according to various embodiments. The thickness of the film shown in FIG. 7C is substantially equal to the thickness of the film shown in FIG. 7B.

The experiment proved that there were hardly wrinkles for the gold film prepared using the pre-biaxial strain method. Wrinkles may still form for gold film thickness as large as 500 nm by using the method according to various embodiments, which may be due to the good adhesion and the special dynamic mingling process.

The wrinkled structure may also contribute to the stretchability of the film. FIG. 8A shows a scanning electron microscopy (SEM) image of the stretchable electrode according to various embodiments under tensile strain of 50%, while the inset shows a magnified image of the upheaval blocks remain connected.

As shown in FIG. 8A, initial cracks may appear at the depression positions and may be then propagated. Some upheaval blocks may be twisted and may still be connected, thus contributing to the stretchability of the film.

FIG. 8B shows a further scanning electron microscopy (SEM) image of the stretchable electrode according to various embodiments illustrating the connecting effect by twisting of the upheavals. FIG. 8C shows another scanning electron microscopy (SEM) image of the stretchable electrode according to various embodiments illustrating the connecting effect by twisting of the upheavals.

FIG. 8D shows a scanning electron microscopy (SEM) image of the stretchable electrode according to various embodiments under tensile strain of 30%. The electrode shown in FIG. 8D may be prepared with a ratio of cross linker to monomer of 1 : 5 and pre-cured for a duration of 25 minutes before deposition. FIG. 8E shows a magnified image of the stretchable electrode shown in FIG. 8D according to various embodiments.

FIG. 8F shows a scanning electron microscopy (SEM) image of the stretchable electrode according to various embodiments under tensile strain of 30%. The electrode shown in FIG. 8F may be prepared with a ratio of cross linker to monomer of 1 : 10 and pre-cured for a duration of 25 minutes before deposition. FIG. 8G shows a magnified image of the stretchable electrode shown in FIG. 8F according to various embodiments.

However, not all upheaval blocks may be connected within the cracks. The stretchability achieved by films was surprisingly good (> 130%) and may not fully be accounted for by the upheaval blocks. The experiments carried out point to another dominating mechanism to explain the high stretchability of the electrode according to various embodiments.

FIG. 8H is a plot of resistance (in ohms or $\Omega$) as a function of strain (in percent or %) showing the resistance change of the stretchable electrode according to various embodiments under varying strain, with the inset showing an optical image of the test setup. The resistance is out of the measurement range at around 135%.

FIG. 8H points to another dominating mechanism that may contribute to the stretchability achieved by films. Close observations of FIGS. 8A, D-G show numerous randomly distributed micro cracks. The strain energy may be released by these micro cracks which form a connecting network in the PDMS layer. The strain regulation effect induced by the underlying micro pillars may be crucial.

Simulation was carried out to investigate the importance of the micro pillars. FIG. 9A is a schematic showing a finite element method (FEM) investigating the strain redistribution mechanism behind the stretchability of the stretchable electrode according to various embodiments. FIG. 9B is a schematic showing the finite element method (FEM) includes randomly oriented micro pillars in the simulation of the stretchable electrode according to various embodiments. As shown in FIGS. 9A-B, a model is built based on a suspended film supported by micro pillars placed randomly under the suspended film. FIG. 9C shows the simulation results of the finite element method (FEM) investigating the strain redistribution mechanism behind the stretchability of the stretchable electrode according to various embodiments. FIG. 9D shows the simulation of strain regulation effect induced by the micro pillars according to various embodiments.

The simulation results indicate that the strain distribution may indeed be regulated by the pillars under the suspended film. The strain distribution may determine the crack formation during stretching and the random distribution may significantly benefit the stretchability.

The randomly distributed micro cracks may increase the resistance of the film, thus allowing the stretchable conductor to be used as a stretchable strain sensor for detecting or measuring the mechanical deformation is applied in the film. The sensitivity is reflected by the gauge factor defined as:

$$\text{Gauge factor} = \frac{\Delta R}{R_0 \varepsilon} \qquad\qquad (2)$$

where $\varepsilon$ is the strain, $R_0$ is the unstrained resistance of the gauge, and $\Delta R$ is the change in strain gauge resistance. The gauge factor achieved may be as large as 20.

FIG. 9E is a plot of fractional change of resistance as a function of the number of cycles illustrating the resistance change of the stretchable electrode according to various embodiments under cyclic tensile strain. FIG. 9E shows that the resistance change may be repeatable at least for 1,000 strain cycles.

The effects of the ratio of cross linker to monomer as well as pre-cured time on the stretchability and gauge factor were also investigated in detail. FIG. 9F is a plot of stretchability (in percent or %) as a function of pre-curing time / ratio of cross linker to monomer used according to various embodiments. The ratio of cross-linker to monomer used for the pre-curing time experiment is 1 : 10. The pre-curing time for the ratio experiment is 25 minutes. The stretchability decreases with the decrease of the ratio of cross linker to monomer used, and/or the decrease of the pre-curing time. This indicates that initially less cured PDMS that is less viscous, i.e. films formed with a smaller ratio of cross-linker to monomer or shorter pre-curing time, may possess poor stretchability. The liquid with less viscosity may tend to be discontinuous during the stretching.

A pre-cured sample having a particular range or value of viscosity, which is determined by a certain range or value of pre-curing time, may form a suspended composite film having maximum stretchability. Pre-curing times longer or shorter than this range or value may result in a composite film having less stretchability. For example, maximum stretchability may be obtained by samples pre-cured with 25 minutes of pre-curing time.

For less viscous samples, the number of the pulled-out micro pillars may be decreased dramatically, which lose the ability of strain regulation, resulting in the concentration of strain and causing poor stretchability.

The stretchability may also decrease with thinner gold film formed. The heat transfer decreases when forming a thinner gold film, which may be equivalent to less pre-curing. However, when the gold film thickness increases beyond a point, the wrinkled structure may become relatively filled up. Also, the larger thickness may increase the defects inside the film and may also lower the ductility of the film. Accordingly, the stretchability may increase with gold film thickness up to a point, and may decrease with a further increase in gold film thickness after the point. Additionally, since sensitivity behaves in a reverse manner to stretchability, sensitivity shows a reverse change trend, i.e. sensitivity may decrease and may then increase with increasing gold film thickness. FIG. 9G is a plot of stretchability (in percent or %) /gauge factor as a function of film thickness (in nanometers or nm) according to various embodiments.

Adhesion performance may also be vital for various applications of stretchable electronics. FIG. 9H is a plot of adhesion strength (in megaPascals or MPa) as a function of pre-curing time / ratio of cross linker to monomer used according to various embodiments.

FIG. 9H shows that the adhesion strength achieved may be as large as about 2 MPa, which may be much greater than the adhesion strength of a normal crack-based stretchable gold film (NCSG) ($\sim$ 0.2 MPa) or the adhesion strength of a flat non-stretchable gold film (FNSG) ($\sim$ 0.5 MPa). The ratio of cross-linker to monomer used for the pre-curing time experiment is 1 : 10. The pre-curing time for the ratio experiment is 25 minutes. Both the NCSG film and the FNSG film are formed with 10 nm chromium as the adhesion layer.

FIGS. 9F and 9H show that the 1 : 10 sample demonstrates the highest stretchability and adhesion strength when pre-cured for 25 minutes. It may be expected that the optimized pre-curing time would be different for other ratios. The optimized pre-curing time may be affected by factors such as the ratio, the precursors used, or the curing temperature.

In order to intuitively demonstrate the adhesion enhancement, high-adhesion Kapton tape may be employed to do a comparison peel test. FIG. 10 shows (a) an image showing adhering a Kapton tape to a flat non-stretchable gold film, (b) an image showing peeling the Kapton tape from the flat non-stretchable gold film, (c) an image showing a portion of the flat non-stretchable gold film being peeled off, (d) an image showing adhering a Kapton tape to a normal crack-based stretchable gold film, (e) an image showing peeling the Kapton tape from the normal crack-based stretchable gold film, (f) an image showing a portion of the normal crack-based stretchable gold film being peeled off, (g) an image of adhering a Kapton tape to a stretchable electrode according to various embodiments, (h) an image showing peeling of the Kapton tape from the stretchable electrode according to various embodiments, (i) an image showing a smaller portion of the stretchable electrode according to various embodiments being peeled off, and (j) an image of a multimeter to measure the electrical conductivity of the stretchable electrode according to various embodiments after the peel test in (g) - (i).

It was clearly shown that the high-adhesion Kapton tape may effect little damage to the stretchable electrode according to various embodiments, but may peel a large area of the normal stretchable gold film and non-stretchable gold film. The good adhesion achieved may be due to the dynamic mingling process as discussed before. The hot high-speed gold nanoparticles may strike the non-fullycured polymer and may thus be fully inter-connected with the polymer.

FIG. 11 shows (a) a scanning electron microscopy image (SEM) showing the mingling effect of the evaporated gold and the polydimethylsiloxane (PDMS) according to various embodiments, and (b) a magnified image of the boxed region indicated in (a).

The force may be increased until the suspended layers are separated from the PDMS base layer. The bottom of the gold film may be exposed, which may indicate that the fracture is induced by interface failure, even though the mingling effects have already significantly enhanced the adhesion. The fracture strength of a PDMS film under tensile strength is around 2.24 MPa, while the adhesion strength of the stretchable electrode may be around 2 MPa, which is comparable to the fracture strength of the PDMS film. This indicates the adhesion strength has greatly improved and may be almost reaching the limit.

In order to study the fracture mechanism, the surface morphology of both the top of the PDMS base layer and the bottom of the suspended layers were examined.

FIG. 12A is a scanning electron microscopy (SEM) image showing the surface morphology of the bottom of the suspended layers according to various embodiments. FIG. 12B is a magnified scanning electron microscopy (SEM) image of a region shown in FIG. 12A according to various embodiments.

FIG. 12C is a scanning electron microscopy (SEM) image showing the surface morphology of the polydimethylsiloxane (PDMS) base layer according to various embodiments. FIG. 12D is a magnified scanning electron microscopy (SEM) image of a region shown in FIG. 12C according to various embodiments.

It may be observed from FIGS. 12C-D that the micro pillars are left on the substrate, indicating that each micro pillar may be fractured or broken at a junction between the pillar and the suspended PDMS film.

Large area suspended double-layer composite suspended films may also be formed. FIG. 13A is an image showing a large stretchable electrode according to various embodiments. FIG. 13B is another image of a stretchable electrode according to various embodiments. Electrodes having an area of about 10 cm by 20 cm have been fabricated. The fabricated electrodes may be ultra-thin, with a thickness as low as 15 $\mu$m.

Various embodiments relate to large area stretchable conductors with high adhesion, which may be fabricated in a simple manner, and which have huge potential in the practical applications such as implantable soft electrodes, body-surface electrophysiology signal detection and other stretchable smart sensors. FIG. 13C is an image of fully encapsulated stretchable electrodes according to various embodiments. FIG. 13D is an image showing the stretchability of encapsulated stretchable electrodes according to various embodiments. The electrodes may be used for wirebonding and the stretchability of the electrodes may be advantageous for such applications.

The stretchable conductors have also been demonstrated as neural stimulators on rats. Stimulation voltages have been applied to drive the movement of thigh muscles of rats, indicating that the stretchable conductors may be used as the neural stimulation electrodes for neural regeneration and rehabilitation.

Various embodiments may have a larger equivalent surface area and a lower impedance as a result of the wrinkled structure.

The impedance of the stretchable electrode may be monitored. The fitting model may be formed by employing the typical constant phase element (CPE) to obtain the equivalent capacitance per area. FIG. 14A shows a plot of impedance (in ohms or $\Omega$) as a function of frequency (in hertz or Hz) of a stretchable electrode according to various embodiments while the inset shows the fitting model.

FIG. 14B is a plot of normalized capacitance comparing the capacitances of a flat gold film (normalized as 1), a first stretchable electrode (formed with a ratio of cross linker to monomer of 1 : 10) according to various embodiments, and a second stretchable electrode (formed with a ratio of cross linker to monomer of 1 : 20) according to various embodiments.

The interface capacitance may at least be 4 times larger than that of the flat gold film. The capacitance may also be easily tunable by adjusting the ratio of cross linker to monomer. The ratio of cross linker to monomer may have an effect on capacitance as different ratios result in films with different densities of the wrinkles as discussed earlier.

Stretchable electrodes may be implanted and entwined around the tracts of sciatic nerve in thigh muscles of rats as nerve stimulators. The induced electromyography (EMG) signal may be monitored simultaneously. FIG. 15A shows (left) a schematic showing the implantation of the stretchable electrode according to various embodiments in a rat to stimulate the sciatic nerve, and (right) an image showing the implantation of the electrode according to various embodiments. FIG. 15B is an image showing the recording of the electromyography (EMG) signal and the providing of the stimulation signal using the stretchable electrodes according to various embodiments. FIG. 15C is another image showing the rat implanted with the stretchable electrodes according to various embodiments, with the inset showing an X-ray image of the implanted electrode within the rat.

Both stimulation and EMG detection may be carried out. FIG. 15D is a plot of voltage (in millivolts or mV) as a function of time (in milliseconds or ms) showing the electromyography (EMG) signal detected and the stimulation signal applied to the electrode according to various embodiments. FIG. 15E shows a magnification of the electromyography (EMG) signal shown in FIG. 15D. FIGS. 15D-E show clear inputs and outputs. FIG. 15F is a plot of peak-to-peak value of the induced electromyography (EMG) signal as a function of the simulation voltage applied to the stretchable electrode according to various embodiments. As shown in FIG. 15F, the EMG response is more sensitive at the early stage of the stimulation and becomes less sensitive with the increase in applied stimulation voltage (measured in terms of the peak-peak value of the response).

With further surface modification and treatment, the stretchable electrodes may be used as long-term implants to monitor/stimulate the target nerve, due to the superior stretchability, stability, biocompatibility, large surface area and/or high adhesion.

Various embodiments may have huge potential in implantable stretchable electrodes and/or other bio-signal detection electrodes, due to the simple fabrication process and good performance of the obtained stretchable electrodes. Various embodiments may be easily fabricated in large scale. Various embodiments may include gold as the conductive material, and PDMS as the polymer. Gold is biocompatible and PDMS is cheap and commonly found, which may result in a relatively low price per area of the electrode.

**Claims**

1. A method of forming a stretchable electrode (200), the method comprising:

   providing a non-fully cured sample on a substrate; and
   depositing a metal on the non-fully cured sample via thermal evaporation to form the stretchable electrode (200);
   wherein the non-fully cured sample is uncured or is partially cured for a duration of less than 30 minutes;
   wherein the partially cured sample is formed by heating at a temperature of less than 70 °C;
   wherein the stretchable electrode (200) comprises:

      a base layer (202);
      a wrinkled layer (204);
      a plurality of pillars (206) extending from the base layer (202) to the wrinkled layer (204); and
      a metal film (208) on the wrinkled layer (204), the metal film (208)
      comprising the metal; and

   wherein the base layer (202), the wrinkled layer (204), and the plurality of pillars (206) comprise polydimethylsiloxane.

2. The method according to claim 1,
   wherein the uncured sample comprises a monomer and a cross-linker.

3. The method according to claim 1 or claim 2,
   wherein the partially cured sample comprises a plurality of polymeric chains joined to another via one or more cross-links.

**4.** The method according to any one of claims 1 to 3,
wherein the non-fully cured sample is partially cured for a duration of less than 26 minutes.

**5.** The method according to any one of claims 1 to 4,
wherein the non-fully cured sample is partially cured for a duration of less than 15 minutes.

**6.** The method according to any one of claims 1 to 5, the method further comprising:
providing a metal source comprising the metal.

**7.** The method according to any one of claims 1 to 6,
wherein the deposition of the metal on the non-fully cured sample is carried out in a vacuum.

**8.** The method according to claim 7,
wherein the vacuum is at $1.0 \times 10^{-6}$ Torr.

**9.** The method according to any one of claims 1 to 8,
wherein the metal is any one selected from a group consisting of gold, palladium, aluminium and copper.

**10.** The method according to any one of claims 1 to 9,
wherein the non-fully cured sample is fully cured during deposition of the metal.

**11.** A stretchable electrode (200) comprising:

a base layer (202);
a wrinkled layer (204); and
a plurality of pillars (206) extending from the base layer (202) to the wrinkled layer (204); and
a metal film (208) on the wrinkled layer (204), the metal film (208) comprising a metal;
wherein the base layer (202), the wrinkled layer (204), and the plurality of pillars (206) comprise polydimethylsiloxane.


**Patentansprüche**

**1.** Verfahren zum Ausbilden einer dehnbaren Elektrode (200), wobei das Verfahren aufweist:

Bereitstellen einer nicht vollständig gehärteten Probe auf einem Substrat und
Abscheiden eines Metalls auf der nicht vollständig gehärteten Probe durch thermische Verdampfung, um die dehnbare Elektrode (200) auszubilden,
wobei die nicht vollständig gehärtete Probe ungehärtet ist oder für eine Dauer von weniger als 30 Minuten teilweise gehärtet wird,
wobei die teilweise gehärtete Probe durch Erwärmen bei einer Temperatur von weniger als 70 °C ausgebildet wird,
wobei die dehnbare Elektrode (200) aufweist:

eine Basisschicht (202),
eine Faltenschicht (204),
eine Mehrzahl von Säulen (206), die sich von der Basisschicht (202) zu der Faltenschicht (204) erstrecken, und
einen Metallfilm (208) auf der Faltenschicht (204), wobei der Metallfilm (208) das Metall aufweist, und

wobei die Basisschicht (202), die Faltenschicht (204) und die Mehrzahl von Säulen (206) Polydimethylsiloxan aufweisen.

**2.** Verfahren gemäß Anspruch 1,
wobei die ungehärtete Probe ein Monomer und einen Vernetzer aufweist.

**3.** Verfahren gemäß Anspruch 1 oder Anspruch 2,
wobei die teilweise gehärtete Probe eine Mehrzahl von Polymerketten aufweist, die über eine oder mehrere Vernetzungen miteinander verbunden sind.

**4.** Verfahren gemäß irgendeinem der Ansprüche 1 bis 3,
wobei die nicht vollständig gehärtete Probe für eine Dauer von weniger als 26 Minuten teilweise gehärtet wird.

**5.** Verfahren gemäß irgendeinem der Ansprüche 1 bis 4,
wobei die nicht vollständig gehärtete Probe für eine Dauer von weniger als 15 Minuten teilweise gehärtet wird.

**6.** Verfahren gemäß irgendeinem der Ansprüche 1 bis 5, wobei das Verfahren ferner aufweist:
Bereitstellen einer Metallquelle, die das Metall aufweist.

**7.** Verfahren gemäß irgendeinem der Ansprüche 1 bis 6,
wobei das Abscheiden des Metalls auf die nicht vollständig gehärtete Probe in einem Vakuum durchgeführt wird.

**8.** Verfahren gemäß Anspruch 7,
wobei das Vakuum bei $1{,}0 \times 10^{-6}$ Torr liegt.

**9.** Verfahren gemäß irgendeinem der Ansprüche 1 bis 8,
wobei das Metall irgendeines ist, das aus einer Gruppe bestehend aus Gold, Palladium, Aluminium und Kupfer ausgewählt wird.

**10.** Verfahren gemäß irgendeinem der Ansprüche 1 bis 9,
wobei die nicht vollständig gehärtete Probe während des Abscheidens des Metalls vollständig gehärtet wird.

**11.** Dehnbare Elektrode (200), aufweisend:

eine Basisschicht (202),
eine Faltenschicht (204) und
eine Mehrzahl von Säulen (206), die sich von der Basisschicht (202) zu der Faltenschicht (204) erstrecken, und
einen Metallfilm (208) auf der Faltenschicht (204), wobei der Metallfilm (208) ein Metall aufweist,
wobei die Basisschicht (202), die Faltenschicht (204) und die Mehrzahl von Säulen (206) Polydimethylsiloxan aufweisen.

## Revendications

**1.** Procédé destiné à former une électrode étirable (200), le procédé comprenant les étapes suivantes :

fournir un échantillon non totalement durci sur un substrat ; et
déposer un métal sur l'échantillon non totalement durci par l'intermédiaire d'une évaporation thermique afin de former l'électrode étirable (200) ;
dans lequel l'échantillon non totalement durci est non durci, ou est durci en partie, pendant une durée inférieure à 30 minutes ;
dans lequel l'échantillon durci en partie, est formé par chauffage à une température inférieure à 70 °C ;
dans lequel l'électrode étirable (200) comprend :

une couche de base (202) ;
une couche plissée (204) ;
une pluralité de montants (206) qui s'étendent à partir de la couche de base (202) vers la couche plissée (204) ; et
une couche métallique (208) sur la couche plissée (204), la couche métallique (208) comprenant le métal ; et

dans lequel la couche de base (202), la couche plissée (204), et la pluralité de montants (206), comprennent du polydiméthylsiloxane.

**2.** Procédé selon la revendication 1,
dans lequel l'échantillon non durci, comprend un monomère et un agent de réticulation.

3. Procédé selon la revendication 1 ou la revendication 2, dans lequel l'échantillon durci en partie, comprend une pluralité de chaînes polymères jointes les unes aux autres par l'intermédiaire d'une ou de plusieurs réticulations.

4. Procédé selon l'une quelconque des revendications 1 à 3, dans lequel l'échantillon non totalement durci, est durci en partie pendant une durée inférieure à 26 minutes.

5. Procédé selon l'une quelconque des revendications 1 à 4, dans lequel l'échantillon non totalement durci, est durci en partie pendant une durée inférieure à 15 minutes.

6. Procédé selon l'une quelconque des revendications 1 à 5, le procédé comprenant en outre l'étape suivante : fournir une source de métal comprenant le métal.

7. Procédé selon l'une quelconque des revendications 1 à 6, dans lequel le dépôt du métal sur l'échantillon non totalement durci, est exécuté sous vide.

8. Procédé selon la revendication 7, dans lequel le vide est égal à $1,0 \times 10^{-6}$ torr.

9. Procédé selon l'une quelconque des revendications 1 à 8, dans lequel le métal est l'un quelconque sélectionné dans le groupe constitué par l'or, le palladium, l'aluminium et le cuivre.

10. Procédé selon l'une quelconque des revendications 1 à 9, dans lequel l'échantillon non totalement durci, est entièrement durci au cours du dépôt du métal.

11. Électrode étirable (200) comprenant :

   une couche de base (202) ;
   une couche plissée (204) ; et
   une pluralité de montants (206) qui s'étendent à partir de la couche de base (202) vers la couche plissée (204) ; et
   une couche métallique (208) sur la couche plissée (204), la couche métallique (208) comprenant un métal ;
   dans laquelle la couche de base (202), la couche plissée (204), et la pluralité de montants (206) comprennent du polydiméthylsiloxane.

**FIG. 1**

Provide a non-fully cured sample on a substrate

102

Deposit a metal on the non-fully cured sample via thermal evaporation to form the stretchable electrode

104

**FIG. 2**

200

base layer

202

a plurality of pillars

206

wrinkled layer

204

a metal film

208

**FIG. 3A**

Dynamic interface mingling

**FIG. 3B**

Physical modeling

**FIG. 3C**

Simulation results

FIG. 3D

rigid backing

gold

Cured
PDMS

base layer

Cured PDMS

wrinkled layer    pillars

Cross-section diagram

FIG. 4A

## FIG. 4B

Cross-section view

Gold film
PDMS film
PDMS micropillars

PDMS substrate

1 µm

FIG. 4C

500 nm

**FIG. 4D**

FIG. 4E

FIG. 4F

2 μm

μm
1.20
0.55
-0.10
-0.75
-1.40

## FIG. 4G

EP 3 545 571 B1

FIG. 4H

**FIG. 5A**

**FIG. 5B**

**FIG. 5C**

**FIG. 5D**

**FIG. 5E**

**FIG. 5F**

FIG. 6A

**FIG. 6B**

**FIG. 6C**

**FIG. 6D**

**FIG. 6E**

**FIG. 6F**

FIG. 7A

**FIG. 7B**

**FIG. 7C**

FIG. 8A

FIG. 8B

**FIG. 8C**

**FIG. 8D**

**FIG. 8E**

## FIG. 8F

50 µm

**FIG. 8G**

FIG. 8H

EP 3 545 571 B1

**FIG. 9A**

**FIG. 9B**

**FIG. 9C**

Strain

0.68    0.45    0.23    0.002

**FIG. 9D**

+6.77e−01
+5.65e−01
+4.52e−01
+3.39e−01
+2.27e−01
+1.14e−01
+1.25e−03

**FIG. 9E**

**FIG. 9F**

**FIG. 9G**

**FIG. 9H**

FIG. 10

FIG. 11

EP 3 545 571 B1

**FIG. 12B**

b  Bottom of suspended film

1 μm

EP 3 545 571 B1

FIG. 12C

EP 3 545 571 B1

FIG. 12D

FIG. 13A

## FIG. 13B

FIG. 13C

**FIG. 13D**

**FIG. 14A**

FIG. 14B

## FIG. 15A

Reference electrodes

sciatic nerve

Stimulation electrodes

EMG detecting needles

FIG. 15B

# FIG. 15C

FIG. 15D

0.12 mV

2 s

EMG signal

0.3 mV

2 s

Stimulation signal

**FIG. 15E**

**FIG. 15F**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- SG 10201609888R

- US 20150345025 A1

**Non-patent literature cited in the description**

- **BOWDEN N et al.** Spontaneous formation of ordered structures in thin films of metals supported on an elastomeric polymer. *NATURE,* 14 May 1998, vol. 393, 146-149